## Europäisches Patentamt

(19) **European Patent Office**

**Office européen des brevets**

(11) Publication number : **0 357 637 B1**

## (12) EUROPEAN PATENT SPECIFICATION

(45) Date of publication of patent specification :
**17.06.92 Bulletin 92/25**

(21) Application number : **88903494.8**

(22) Date of filing : **04.03.88**

(86) International application number :
**PCT/US88/00568**

(87) International publication number :
**WO 88/06595 07.09.88 Gazette 88/20**

(51) Int. Cl.[5] : **C07K 7/08, G01N 33/574, A61K 37/02, C07K 1/02, G01N 33/48**

(54) GENERAL CANCER-ASSOCIATED SCM-RECOGNITION FACTOR, PREPARATION AND METHOD OF USE.

(30) Priority : **06.03.87 US 22759**

(43) Date of publication of application :
**14.03.90 Bulletin 90/11**

(45) Publication of the grant of the patent :
**17.06.92 Bulletin 92/25**

(84) Designated Contracting States :
**AT BE CH DE FR GB IT LI LU NL SE**

(56) References cited :
**Chemical Abstracts, volume 81, no. 15, 14 October 1974, (Columbus, Ohio, US), L. Cercek et al.: "Biophysical differentiationbetween normal human an dchronic lymphocytic leukemia lymphocytes", see page 314, abstract 89562c, & Proc. Leucocyte Cult.Conf. 1973 (Pub. 1974), 8, 553-8**
**Biological Abstracts, volume 71, no. 1, 1981, (Philadelphia, PA., US), N.D. Schnuda: "Evaluation of fluroescence polarizationof human blood lymphocytes (SCM structuredness of cytoplasmic matrix test) in the diagnosis of cancer", see page 425, abstract4093, & Cancer (Phila) 46(5): 1164-1173**
**Biological Abstracts, volume 62, 15 October 1976, (Philadelphia, PA., US), L. Cercek et al.: "Changes in the structurednessof cytoplasmic matrix (SCM) in hum lymphocytes induced by PHA and cancer basic protein as measured in single cells", see page 4375,abstract 44333, & Br. J. Cancer 33 (5): 539-543, 1976**
**Biological Abstracts, volume 60, no. 4, 1975, (Philadelphia, PA., US), L. Cercek et al.: "Apparent tumour specificity withthe SCM test", see pages 2262-2263, abstract 21127, & Br. J. Cancer 31(2): 252-263, 1975**
**Biological Abstracts, volume 81, no. 5, 1986, (Philadelphia, PA., US), J. Matsumoto et al.: "Clinical evaluation offluorescein polarization of peripheral lymphocytes (SCM test) in the diagnosis of cancer", see page AB-687, abstract 45158, &J. Jpn. Soc. Cancer Ther. 20(4): 728-734, 1985**
**Biological Abstracts, volume 81, no. 5, 1986, (Philadelphia, PA., US), S. Chaitchik et al.: "Tumor specificity of thestructuredness of cytoplasmic matrix test for cancer diagnosis", see pages AB-666-AB-667, abstract 44969, & Eur. J. CancerClin. Oncol. 21(10): 1165-1170, 1985**

(56) References cited :
**Biological Abstracts, volume 69, no. 7, 1980, (Philadelphia, PA., US), H. Orjasaeter et al.: "Response of thymus-derivedlymphocytes to phytohemagglutinin and cancer tissueassociated antigens, measured by the intracellular fluorescence polarizationtechnique (cytoplasmic matric test)", see page 4873, abstract 45479, & Br. J. Cancer 40(4): 628-633, 1979**

(73) Proprietor : **CERCEK, Boris**
**4318 Camphor Avenue**
**Yorba Linda, CA 92686 (US)**
Proprietor : **CERCEK, Lea**
**4318 Camphor Avenue**
**Yorba Linda, CA 92686 (US)**

(72) Inventor : **CERCEK, Boris**
**4318 Camphor Avenue**
**Yorba Linda, CA 92686 (US)**
Inventor : **CERCEK, Lea**
**4318 Camphor Avenue**
**Yorba Linda, CA 92686 (US)**

(74) Representative : **Goldin, Douglas Michael et al**
**J.A. KEMP & CO. 14, South Square Gray's Inn London WC1R 5EU (GB)**

EP 0 357 637 B1

## Description

BACKGROUND OF THE INVENTION

Many diseases occurring in humans and animals can be detected by the presence of foreign substances, particularly in the blood, which are specifically associated with a disease or condition. Tests for antigens or other such substances produced as a result of such diseases show great promise as a diagnostic tool for the early detection of the particular disease which produced the antigen or other substance. Procedures for the detection of such substances must be reliable, reproducible, and sensitive in order to constitute a practical diagnostic procedure for health care providers. In addition, any such procedure should be able to be carried out quickly and inexpensively by persons of ordinary skill and training in laboratory procedures.

For example, in the treatment of the various malignancies that afflict humans and animals, referred to generally as cancer, it is recognized that early detection is the key to effective treatment, especially as most therapeutic procedures are more effective and safer in relatively early stages of cancer than in later stages. For example, many chemotherapeutic drugs that are toxic to malignant cells are also toxic to normal cells, and the higher doses required to cure or arrest more advanced cases of cancer can cause uncomfortable and serious side effects. Also, surgery is most often effective only before the disease has spread or metastasized. Far too many cases of cancer are only discovered too late for effective treatment.

Accordingly, there has been and continues to be a great need for reliable tests that can diagnose cancer at early stages, and a great deal of research effort has gone to this end. In this connection new tests and procedures are being developed to effect early diagnosis of cancer.

One extremely desirable aspect of such a test is its ability either to detect all types of cancer generally, or to detect specific types of cancer, depending on the materials used. The former application of such a test is very important in mass screenings of large patient populations, as would be done in routine checkups. In such mass screenings a test dependent on a particular type of cancer would not be desirable, as there are literally hundreds, if not thousands, of types of cancer and a test that could spot only one or a few types of the disease is far too likely to miss many cases of cancer. In general, these patients would present either no symptoms or vague generalized symptoms that could not be readily linked to a particular type of cancer, so there would be no basis for suspecting a particular type and administering a test specific for that type.

In contrast, once the presence of malignancy is known or strongly suspected, it would be desirable to have a test that could pinpoint the particular type of malignancy present. Such a test could add greatly to the efficiency of treatment, because many of the most effective cancer therapies, such as chemotherapeutic agents, are only effective against one type of cancer or at best, a narrow range of types, and the wrong chemotherapy can do more harm than good.

In an effort to meet this need and to improve the diagnosis and early detection of cancer in human and animal bodies, a test procedure has been developed which involves the measurement of changes in the structuredness of the cytoplasmic matrix (SCM) of living lymphocytes when exposed either to phytohaemagglutinin or to cancer-associated antigens. This procedure has been described in L. Cercek, B. Cercek, and C.I.V. Franklin, "Biophysical Differentiation Between Lymphocytes from Healthy Donors, Patients with Malignant Diseases and Other Disorders," Brit J. Cancer 29, 345-352 (1974), and L. Cercek and B. Cercek, "Application of the Phenomenon of Changes in the Structuredness of Cytoplasmic Matrix (SCM) in the Diagnosis of Malignant Disorders: a Review," Europ. J. Cancer 13, 903-915 (1977).

In accordance with this procedure, a subpopulation of potentially SCM-responding lymphocytes is separated from a blood sample of the patient being tested and the lymphocytes are incubated with malignant tissue or extracts of malignant tissue. If the blood sample donor is afflicted with a malignancy, there will be a characteristic SCM response that can be differentiated from the SCM response of lymphocytes from donors not afflicted with a malignancy. The SCM response is determined by measuring changes in intracellular fluorescein fluorescence polarization of the SCM-responding lymphocytes.

The changes seen in the SCM test are believed to reflect changes in the internal structure of the lymphocyte as the lymphocyte is activated for synthesis. These changes are seen as a decrease in the fluorescence polarization of the cells when polarized light is used. to excice the fluorescein present in the cells. Fluorescence polarization is a measure of intracellular rigidity; the greater the intracellular mobility, the less the measured fluorescence polarization. An observed decrease in fluorescence polarization is thought to result mainly from changes in the conformation of the mitochondria, the energy-producing organelles of the cell. The change in the mitochondria is believed to result from the contractions of the cristae or inner folds of the mitochondrial membrane. The SCM reflects the forces of interaction between macromolecules and small molecules such as water molecules, ions, adenosine triphosphate, and cyclic adenosine phosphate. Perturbations of these interactions result in changes in the SCM.

The SCM test is capable of responding to a relatively small quantity of malignant cells. About $10^9$ cells in a person weighing 70 kg are enough to cause the lymphocytes to respond in the SCM test in the characteristic pattern of malignancy. In mice, when as few as $7.5 \times 10^5$ Ehrlich ascites (tumor) cells are implanted, the pattern of the response in the SCM test is altered; response to cancer-specific antigens is induced, while the normal response to phytohaemagglutinin in virtually eliminated (L. Cercek and B. Cercek, "Changes in SCM-Responses of Lymphocytes in Mice After Implantation with Ehrlich Ascites Cells," Europ. J. Cancer 17, 167-171 (1981)).

The SCM test allows early detection of cancer, often much earlier than is possible by conventional methods, with relatively little discomfort to the patient except as may be involved in taking a blood sample.

However, this procedure does have disadvantages. For example, it requires preparation of crude extracts from tumor tissues and the like or the use of the tumor tissue itself as a source of cancer-associated antigens. There are several major problems with the use of malignant tissue or extracts of such tissue in the SCM test. It is sometimes difficult to obtain the required quantity of tissue. Also, the use of whole tissues or crude extracts of tissues can introduce interfering substances into the test procedure. These interfering substances can adversely affect the sensitivity of the test or adversely affect the test results themselves. The presence or absence of these interfering substances can easily vary from batch to batch of malignant tissue, introducing undesirable variability into the SCM test. Additionally, because the interfering substances are present in whole tissue or crude extracts, they are very difficult to identify or quantitate.

Accordingly it is very desirable to identify, separate, and purify the factor or factors that provoke a response by SCM-responding lymphocytes. The use of such purified factor or factors would enhance the SCM cancer screening test because interfering substances would not be present, and would aid in the study of cancer, its causes and its effects on human and animal bodies.

## SUMMARY OF THE INVENTION

We have discovered cancer recognition factors in animal and human body fluids, more particularly in blood plasma and urine, which, if the donor body is afflicted with cancer, produce a response in SCM-responding lymphocytes that is identical to the response by such lymphocytes to cancer-associated extracts and/or tumor tissue in the SCM test. As described herein the factors are designated in the singular and referred to as the "general cancer-associated SCM-recognition factor," the "SCM-recognition factor," or merely as the "SCM factor."

The SCM factor is believed to be a peptide or peptide-like material and to comprise several closely related peptides. The SCM factor is believed to include a thirteen-amino-acid fragment having the sequence Phe-$R_1$-Lys-Pro-Phe-$R_2$-Phe-$R_3$-Met-$R_4$-$R_5$-$R_6$-$R_7$, wherein $R_1$ is selected from the group consisting of Asn and Gln, $R_2$, $R_3$, and $R_4$ are each independently selected from the group consisting of Val, Leu, and Ile, $R_6$ is selected from the group consisting of Asp and Glu, and $R_6$ and $R_7$ are each independently selected from the group consisting of Asn and Gln. This sequence does not include the amino terminus of the SCM factor.

The general cancer-associated SCM-recognition factor consists essentially of low molecular weight peptide passing through filters with a nominal 1000-dalton molecular weight cutoff and retained by filters with a nominal 500-dalton molecular weight cutoff, and producing at least a ten percent decrease in the intracellular fluorescence polarization value of SCM-responding lymphocytes isolated from donors afflicted with cancer when used to challenge the lymphocytes in the standard SCM test. The factor purified by the process described below has the approximate amino acid composition of ($Asx_2$, $Glx_3$, Ser, His, $Gly_5$, Thr, Arg, $Ala_3$, Tyr, Met, $Val_3$, $Phe_3$, Ile, $Leu_3$, $Lys_2$).

On the basis of amino acid sequencing of tryptic fragments of the SCM factor, a sixteen-amino-acid segment within one preparation of the factor has the amino acid sequence Phe-Asn-Lys-Pro-Phe-Val-Phe-Leu-Met-Ile-Asp-Gln-Asn-Phe-Ser-Lys, the segment not including the amino terminus of the factor.

Alternatively, a fifteen-amino-acid segment within another preparation of the factor has the amino acid sequence Phe-Asn-Lys-Pro-Phe-Val-Phe-Leu-Met-Ile-Asp-Gln-Asn-Thr-Lys, the segment also not including the amino terminus of the factor.

These two tryptic fragments are both fully active in the SCM test. It is believed that the first thirteen amino acids, which are common to the two fragments, are the most important in producing SCM-activity. Accordingly, an important additional aspect of the invention is an SCM factor comprising only peptides of defined sequence, the peptides either having been shown directly to have SCM activity or believed to have such activity. Most broadly, such a factor comprises substantially only peptides having at least thirteen amino acid residues including a sequence of Phe-$R_1$-Lys-Pro-Phe-$R_2$-Phe-$R_3$-Met-$R_4$-$R_5$-$R_6$-$R_7$ therein, where $R_1$ is selected from the group consisting of Asn and Gln, $R_2$, $R_3$, and $R_4$ are each independently selected from the group consisting of Val, Leu, and Ile, $R_6$ is selected from the group consisting of Asp and Glu, and $R_6$ and $R_7$ are each independently selected from the group consisting of Asn and Gln. Preferably, this factor contains substantially only one peptide.

When the full sequence information from the two tryptic fragments is utilized, it gives rise to two separate and parallel classes of SCM factors, each including the thirteen-amino-acid fragment as defined above.

The first class most broadly comprises substantially only peptides having at least fifteen amino acid residues including a sequence of Phe-$R_1$-Lys-Pro-Phe-$R_2$-Phe-$R_3$-Met-$R_4$-$R_5$-$R_6$-$R_7$-$R_8$-Lys therein, wherein $R_1$ through $R_7$ are as defined above and $R_5$ is selected from the group consisting of Ser and Thr. Preferably this factor contains substantially only one peptide.

Within this class, a more closely defined factor comprises substantially only peptides including a sequence of Phe-Asn-Lys-Pro-Phe-Val-Phe-Leu-Met-Ile-Asp-Gln-Asn-Thr-Lys therein. These peptides can include additional flanking sequences on either side of the specified sequence. Preferably this factor contains substantially only one peptide.

The most closely defined factor within this class comprises a substantially pure peptide consisting essentially of the amino acid sequence Phe-Asn-Lys-Pro-Phe-Val-Phe-Leu-Met-Ile-Asp-Gln-Asn-Thr-Lys.

The second class most broadly comprises substantially only peptides having at least sixteen amino acid residues including a sequence of Phe-$R_1$-Lys-Pro-Phe-$R_2$-Phe-$R_3$-Met-$R_4$-$R_5$-$R_6$-$R_7$-Phe-$R_8$-Lys therein, wherein $R_1$ through $R_7$ are as defined above and $R_5$ is selected from the group consisting of Ser and Thr. Preferably this factor contains substantially only one peptide.

Within this class, a more closely defined factor comprises substantially only peptides including a sequence of Phe-Asn-Lys-Pro-Phe-Val-Phe-Leu-Met-Ile-Asp-Gln-Asn-Phe-Thr-Lys therein. These peptides can include additional flanking sequences on either side of the specified sequence. Preferably this factor contains substantially only one peptide.

The most closely defined factor within this class comprises a substantially pure peptide consisting essentially of the amino acid sequence Phe-Asn-Lys-Pro-Phe-Val-Phe-Leu-Met-Ile-Asp-Gln-Asn-Phe-Thr-Lys. Preferably, this peptide has an activity in the SCM test such that $5 \times 10^{-2}$ femtograms of the peptide produce a decrease in polarization value of at least 30 percent when used to challenge SCM-responding lymphocytes from donors afflicted with cancer.

The SCM factor can be produced by ultrafiltering a body fluid from a donor afflicted with cancer in order to separate a first fraction of the body fluid comprising molecules having an apparent molecular weight greater than 1000 daltons from a second fraction comprising molecules having an apparent molecular weight of less than 1000 daltons. The body fluid is selected from the group consisting of peripheral blood, urine, and plasma. Preferably, after ultrafiltration the factor undergoes a further purification process comprising several stages, each stage resulting in a more highly purified factor.

The first stage of this purification process comprises elution from a gel filtration column with a fractionation range of from 0 to about 700 daltons and capable of separating the salts from the ultrafiltrate, the factor eluting at between about 0.3 and about 0.5 times the total chromatographic bed volume.

The second stage comprises elution from a gel filtration column having a fractionation range of from about 1500 daltons to about 30,000 daltons, the factor eluting from such a column at between about 0.4 and about 0.6 times the total chromatographic bed volume.

The next stage of this purification process comprises elution from an anion-exchange column of diethylaminoethyl cellulose at between about 0.28 M to about 0.31 M of ammonium bicarbonate.

The final stage comprises purifying the factor to substantial homogeneity by reverse-phase high-pressure liquid chromatography.

Although it is preferred to use the more highly purified preparations of the factor in the SCM test, the factor from any stage of the purification, including the initial ultrafiltrate, can be used in the test.

The SCM factor produces a positive response in potentially SCM-responding lymphocytes derived from donors having a variety of different types of malignancies, regardless of the type of cancer present in the donor from whom the SCM factor is isolated; hence the term "general." The factor provokes little or no SCM response in lymphocytes derived from donors free of malignancies. This ability to act as a general cancer-associated antigen makes the factor useful for the general screening of blood samples for the presence of malignancy.

The factor also has other properties of interest. One of these properties is the property of modifying the SCM response of SCM-responding lymphocytes obtained from donors free of malignancy when the factor is contacted with such lymphocytes, whereby the lymphocytes respond in the SCM test to cancer-associated antigens but not to mitogens after the contact. A second property is the property of decreasing the spontaneous in vitro toxicity of potentially SCM-responding lymphocytes toward the human myeloid cell line K 562 by at least 90 percent as measured by $^{51}$Cr release.

Another aspect of the present invention is directed to methods of employing the general cancer-associated SCM factor in the SCM test. Most generally, this method comprises testing lymphocytes from a mammalian donor for the presence or absence of malignancy in the donor by: (1) contacting the lymphocytes with a general cancer-associated SCM-recognition factor; and (2) determining the decrease in the structuredness of the cytoplasmic matrix of the lymphocytes resulting from the step of contacting the suspension of the lymphocytes.

A preferred method for quantifying the decrease in structuredness comprises the steps of: (1) measuring the fluorescence polarization, $P_S$, for an aliquot of the lymphocytes that has been contacted with the general cancer-associated SCM-recognition factor; (2) measuring the fluorescence polarization, $P_C$, for a control aliquot of the lymphocytes that has not been contacted; and (3) determining the ratio of $P_S$ to $P_C$. A ratio of $P_S$ to $P_C$ lower than about 0.9 indicates a positive response to the SCM factor and the presence of a malignancy in the donor of the lymphocytes.

A more preferred method comprises comparing $P_S$ to the fluorescence polarization of another aliquot of the lymphocytes contacted with a mitogen, $P_M$, to determine an SCM response ratio, $RR_{SCM}$, where:

$$RR_{SCM}, = P_S/P_M.$$

An $RR_{SCM}$, of less than about 0.9 indicates the presence of a malignancy in the donor of the lymphocytes.

Alternatively, the method of employing the SCM factor in the SCM test can comprise the steps of: (1) separating potentially SCM-responding lymphocytes from the blood sample; (2) contacting the separated lymphocytes with a general cancer-associated SCM-recognition factor thereby to stimulate the lymphocytes; (3) contacting the stimulated lymphocytes with a fluorogenic agent precursor, defined as a nonfluorogenic compound hydrolyzable intracellularly to a fluorogenic compound, to penetrate the lymphocytes for intracellular enzymatic hydrolysis to the fluorogenic compound, thereby generating stimulated fluor-containing lymphocytes; (4) exciting the stimulated fluor-containing lymphocytes with polarized light thereby causing them to fluoresce; (5) measuring the vertically and horizontally polarized fluorescence emissions from the fluorescing lymphocytes for determining a polarization value for the fluorescing lymphocytes; and (6) comparing the determined polarization value for the stimulated fluor-containing lymphocytes with the polarization value for a control aliquot of lymphocytes from the same donor, thereby to indicate the presence or absence of cancer in the body of the donor of the lymphocytes. Steps (3) and (4) can occur simultaneously.

The lymphocytes can be excited with vertically polarized light. The polarization values when vertically polarized light is used are determined in a fluorescence spectrophotometer in accordance with the relationship:

$$P = \frac{I_V - GI_H}{I_V + CI_H},$$

where, $I_V$ and $I_H$ are the polarized fluorescence intensities in the vertical and horizontal planes, respectively; and G is a correction factor for the unequal transmission of the horizontal and vertical components of the polarized light through the optical system of the spectrophotometer.

Not only does the present invention provide a diagnostic technique for identifying subjects afflicted with cancer, it also comprehends methods for the treatment of such subjects. These methods are based on the observation that the general cancer-associated SCM-recognition factor has the property of decreasing the spontaneous in vitro toxicity of lymphocytes toward malignant cells. In light of that observation, the reduction of the in vivo activity of the SCM-recognition factor should increase the efficiency of immunological surveillance by lymphocytes against malignant cells.

Most generally, this treatment is for a patient at least one of whose body fluids contains a general cancer-associated SCM-recognition factor. The treatment comprises: (1) treating a body fluid containing the SCM-recognition factor to reduce the in vivo effect of the factor by selectively inactivating it; and (2) returning the body fluid to the patient, thereby to enhance the resistance of the patient to the cancer.

In one application of this general method, the body fluid is peripheral blood and the step of treating the body fluid comprises the dialysis of the peripheral blood to remove peptides with an apparent molecular weight of less than 1000 daltons, whereby the SCM-recognition factor is selectively inactivated by its removal from the blood.

In other applications of this general method, the step of treating the body fluid can comprise neutralizing the SCM-recognition factor in the body fluid with an antibody specific for the factor, or with Fab fragments of such an antibody, the Fab fragments being capable of univalent binding of the SCM-recognition factor.

Additionally, the present invention also encompasses a method of imaging cancer cells. This method comprises: (1) labeling an antibody to the general cancer-associated SCM-recognition factor with an imaging substance; and (2) utilizing the labeled antibody to image cancer cells.

The present invention further encompasses a method of directing an anti-cancer substance to cancer cells, comprising: (1) tagging an antibody to the general cancer-associated SCM-recognition factor with the anti-cancer substance thereby to direct the anti-cancer substance to the cancer cells; and (2) administering the tagged antibody to a cancer patient so that the tagged antibody can bind to the cancer cells of the patient.

The present invention still further encompasses a method of determining the level of general cancer-associated SCM-recognition factor in a body fluid using an antibody to the general cancer-associated SCM-recognition factor in an immunoassay. The immunoassay can be a radioimmunoassay, a fluorescence immunoassay, an enzyme-linked immunosorbent assay, or a precipitation immunoassay such as a nephelometric or a turbidimetric assay.

These and other features, aspects, and advantages of the present invention will become better understood from the accompanying description and appended claims.

## DEFINITIONS

Definitions for a number of terms which are used repeatedly in the following Discussion, Examples, and appended claims, are collected here for convenience.

"General":

Nonspecific with respect to the particular type of cancer afflicting either the donor of the body fluid from which the SCM factor of the present invention is purified, or the donor of the lymphocytes used with that factor in the SCM test.

"Fluorogenic Agent Precursor":

A nonfluorogenic compound capable of being taken up by lymphocytes and converted intracellularly by hydrolysis into a fluorogenic compound, of which the example used herein is fluorescein diacetate or FDA.

"Standard SCM Test":

An SCM test using 1.0 ml of a lymphocyte suspension at $6 \times 10^6$ cells/ml and 0.1 ml of the mitogen or antigen, with FDA as the fluorogenic agent precursor and using an excitation wavelength of 470 nm and an emission wavelength of 510 nm for fluorescence polarization measurements.

"Apparent Molecular Weight" and "Nominal Molecular Weight Cutoff":

Both of these terms refer to the fact that the separation of molecules by ultrafiltration according to size is approximate for molecules in the size range of SCM factor, and depends on conformation as well as size. Thus an ultrafilter with a nominal molecular weight cutoff of $x$ daltons will separate molecules with an apparent molecular weight of less than $x$ daltons from molecules with an apparent molecular weight greater than $x$ daltons.

"Substantially Pure General Cancer-Associated SCM-Recognition Factor":

Material exhibiting SCM activity and of such a state of purity that the overwhelming majority of other molecules with specific biological activity, including all proteins and larger peptides, are not present in the material.

"Tryptic Peptide":

A peptide cleaved from a larger peptide by the action of the proteolytic enzyme trypsin, which breaks peptide chains after lysine or arginine residues.

## DESCRIPTION OF THE PREFERRED EMBODIMENTS

This invention relates to our discovery and purification to substantial homogeneity of a peptide that is a general cancer-associated SCM-recognition factor. The factor passes through filters with a nominal molecular weight cutoff of 1000 daltons, but is retained by filters with a nominal molecular weight cutoff of 500 daltons. The factor has an approximate amino acid composition of ($Asx_2$, $Glx_3$, Ser, His, $Gly_5$, Thr, Arg, $Ala_3$, Tyr, Met, $Val_3$, $Phe_3$, Ile, $Leu_3$, $Lys_2$). Two active tryptic fragments have been isolated from different preparations of the factor. These fragments do not include the amino termini of the original factor molecules. The first of these fragments has fifteen amino acids with the sequence Phe-Asn-Lys-Pro-Phe-Val-Phe-Leu-Met-Ile-Asp-Gln-Asn-Thr-Lys. The second of these fragments has sixteen amino acids with the sequence Phe-Asn-Lys-Pro-Phe-Val-Phe-Leu-Met-Ile-Asp-Gln-Asn-Phe-Ser-Lys.

Properties of the factor will be described, including the ability of the factor to modify the SCM response of lymphocytes from donors free of malignancy and to decrease the in vitro cytotoxicity of such lymphocytes toward malignant cells, as well as the crossreactivity of the factor isolated from donors with many different types of cancer. A method for purification of this factor to substantial homogeneity will be described, as well as methods of using this factor in the SCM test. Also to be described will be methods for treatment of cancer patients by reducing the in vivo activity of the factor, a method for imaging cancer cells by using antibody to the factor, and a method for directing anti-cancer substances to cancer cells by tagging antibody to the factor with the anti-cancer substances.

## Properties of the SCM Factor

### 1. Amino Acid Analysis of the SCM Factor

The amino acid composition of the general cancer-associated SCM-recognition factor purified from plasma of patients with breast cancer was determined, and the results are shown in Table 1. Lyophilized samples of the factor purified by RP-HPLC were hydrolyzed for 24 hr at 110°C with 2% thioglycolic acid in 6 N HCl in sealed glass ampules under nitrogen. After hydrolysis the samples were lyophilized. The residues were dissolved with 2% sodium dodecyl sulfate in 0.04 M sodium borate buffer. The amino acids were determined as o-phthaldialdehyde derivatives by their fluorescence after separation by HPLC as described in the article by B.N. Jones, S. Paabo and S. Stein, "Amino Acid Analysis and Enzymatic Sequence Determination of Peptides by an Improved o-Phthaldialdehyde Precolumn Labeling Procedure," J. Liquid Chromat. 4, 565 (1981) and in the manual "Amino Acid Analysis by HPLC," Altex Division, Beckman Instruments, Inc., Berkeley, California 94710.

## TABLE 1

### RELATIVE MOLAR AMINO ACID COMPOSITION OF SCM FACTOR

| Amino Acid | Relative Molar Composition |
|---|---|
| Asx | 2.10 |
| Glx | 3.20 |
| Ser | 1.27 |
| His | 0.65 |
| Gly | 4.60 |
| Thr | 1.11 |
| Arg | 0.86 |
| Ala | 2.60 |
| Tyr | 0.94 |
| Met | 1.58 |
| Val | 3.22 |
| Phe | 3.07 |
| Ile | 0.86 |
| Leu | 2.87 |
| Lys | 1.86 |

The amino acid composition was determined after hydrolysis of dried peptides with 2% thioglycolic acid in 6 N HCl in sealed glass ampules under nitrogen. After hydrolysis samples were lyophilized and the residue dissolved with 2% SDS in 0.04 M sodium borate buffer. The amino acids were determined as o-phthaldialdehyde derivatives by their fluorescence as described in the manual, "Amino Acid Analysis by HPLC," Altex Division, Beckman Instruments, Inc. Berkeley, CA 94710. The amino acids Cys and Pro were not determined.

The amino acid composition data of Table 1 show that the active factor is a peptide. The data are most consistent with a peptide with the approximate amino acid composition (Asx$_2$, Glx$_3$, Ser, His, Gly$_5$, Thr, Arg, Ala$_3$, Tyr, Met$_2$, Val$_3$, Phe$_3$, Ile, Leu$_3$, Lys$_2$).

### 2. Amino Acid Sequence of the SCM Factor

The amino acid sequences of segments isolated from two different preparations of the SCM factor were determined by performing automated Edman degradation procedures using an Applied Biosystems 477A protein sequencer coupled with an on-line 120A PTH-amino acid analyzer. When the entire SCM factor isolated from patients with several types of cancer, such as cancer of the breast, lung, colon, bronchus, or cervix, was subjected to the Edman degradation, no reaction occurred and it was found that the amino-terminus of the SCM factor was blocked. Therefore, in order to determine the sequence of a segment of the molecules, it was necessary to obtain a fragment with a free amino-terminal amino acid. Such fragments were obtained from separate purified preparations of the factor by tryptic digestion followed by purification of the tryptic fragments by reverse phase high pressure liquid chromatography, as detailed below under "Examples." One of these fragments was obtained from a preparation of the factor from plasma of patients with lung cancer, the other from plasma of patients with breast cancer.

In each case a particular fragment, later shown to have SCM activity, eluted at 30.4 volume percent of Phase A, 69.6 volume percent of phase B, in a total volume of about 30 μl. For sequencing, the SCM-active tryptic fragments were directly spotted onto the disc,of the sequencer and subjected to computer-directed automated sequence analysis by successive cycles of Edman degradation, followed by identification of the PTH-amino acids produced thereby in the coupled on-line PTH-amino acid analyzer.

The sequence calling computer program of the sequencer identified a sequence of sixteen amino acids as the sequence of the fragment of the SCM factor isolated from the plasma of lung cancer patients. The sequence was: Phe-Asn-Lys-Pro-Phe-Val-Phe-Leu-Met-Ile-Asp-Gln-Asn-Phe-Ser-Lys. Similarly, the computer program identified a sequence of fifteen amino acids for the fragment of the factor isolated from the plasma of breast cancer patients. The sequence was: Phe-Asn-Lys-Pro-Phe-Val-Phe-Leu-Met-Ile-Asp-Gln-Asn-Thr-Lys.

The first thirteen amino acids in these two tryptic fragments are identical. Although their carboxyl termini are different, if the one missing amino acid, Phe, is inserted in the shorter fragment, the only difference then becomes the substitution of Thr for Ser, a very conservative substitution unlikely to affect the function of the peptide.

### 3. Activity of the SCM Factor in the SCM Test

The purified SCM factor is fully active in the SCM test when used as a challenging agent for lymphocytes isolated from patients with several different types of malignancies. This activity can be demonstrated by assay at any point during the purification of the factor. Details of the results of such assays are given hereinbelow under "Examples." The greatest activity is obtained with material taken through a final purification step, reverse phase high pressure liquid chromatography. One-tenth milliliter of this fraction, having an approximate protein content of 40 picomoles of peptide, causes decreases in intracellular fluorescence polarization of as much as 44.6% when used to challenge SCM-responding lymphocytes isolated from cancer patients, but causes no decrease in intracellular fluorescence polarization when used to challenge the same subpopulation of lymphocytes isolated from healthy donors.

### 4. Activity of the Tryptic Peptides in the SCM Test

Both the fifteen-amino-acid tryptic peptide fragment cleaved from the SCM factor isolated from plasma of breast cancer patients (the breast fragment), and the sixteen-amino-acid tryptic peptide fragments cleaved from the SCM factor isolated from plasma of lung cancer patients (the lung fragment), are fully active in the SCM test. Approximately $5 \times 10^{-2}$ femtograms of the lung fragment (i.e., $5 \times 10^{-17}$ grams, or approximately 16,000 molecules) gave full activity in the SCM test when used as challenging agent for lymphocytes from donors with cancer. The lung fragment reacted equally well with lymphocytes from donors with lung cancer and breast cancer, but caused no response in the SCM test when used to challenge lymphocytes from normal donors. Further details are given hereinbelow under "Examples."

Since the lung fragment and the breast fragment are both fully active in the SCM test and share only the first thirteen amino acids of their sequence, it is believed that this common sequence of thirteen amino acids is the most important sequence in producing SCM activity. Accordingly, a sequence of just these thirteen amino acids, designated herein as the "core," Phe-Asn-Lys-Pro-Phe-Val-Phe-Leu-Met-Ile-Asp-Gln-Asn, is also believed to possess SCM activity.

This sequence is not the only sequence of thirteen amino acids believed to possess SCM activity. It is a well-established principle of protein and peptide chemistry that certain amino acid substitutions, entitled "conservative" amino acid substitutions, can frequently be made in a peptide without altering either the conformation or the function of thatpeptide. Such changes affecting amino acids in the core sequence include Ile and Leu for Val and vice versa, Asp for Glu and vice versa, and Asn for Gln and vice versa. Accordingly, it is believed that altered peptide molecules having the core structure in which any or all of the above-described conservative amino acid substitutions are made exhibit SCM activity and accordingly are considered part of the invention disclosed herein.

The lung and breast fragments are both isolated as part of larger general cancer-associated SCM-recognition molecules. Such fragments apparently can retain their SCM activity when incorporated into larger molecules. Therefore a larger peptide incorporating the thirteen-amino-acid core sequence, with or without the conservative amino acid substitutions discussed above, is also expected to exhibit SCM activity.

The occurrence of these two types of modifications of the core sequence accordingly gives rise to an important additional aspect of the invention. This is an SCM-active factor comprising only peptides of defined sequence, the peptides either having been shown directly to have SCM activity or believed to have such activity. Most broadly such a factor comprises substantially only peptides having at least thirteen amino acid residues including a sequence of Phe-$R_1$-Lys-Pro-Phe-$R_2$-Phe-$R_3$-Met-$R_4$-$R_5$-$R_6$-$R_7$ therein, where $R_1$ is selected from the group consisting of Asn and Gln, $R_2$, $R_3$, and $R_4$ are each independently selected from the group consisting of Val, Leu, and Ile, $R_5$ is selected from the group consisting of Asp and Glu, and $R_5$ and $R_7$ are each independently selected from the group consisting of Asn and Gln. Preferably this factor contains substantially only one such peptide.

When the full sequence information from the lung and breast fragments is utilized, it gives rise to two separate and parallel classes of SCM factors, each including the core sequence or a sequence derived from the core sequence by conservative substitutions. These longer peptides each have one additional possibility for conservative amino acid substitutions, namely Thr for Ser and vice versa. The equivalence of these amino acids is demonstrated by the fact the lung fragment has Ser, while the breast fragment has Thr. The positions of these amino acids in the respective fragments can be aligned by deleting the Phe in position 14 of the lung fragment.

The first class of these factors is derived from the sequence of the breast fragment, Phe-Asn-Lys-Pro-Phe-Val-Phe-Leu-Met-Ile-Asp-Gln-Asn-Thr-Lys. This class most broadly comprises substantially only peptides having at least fifteen amino acid residues including a sequence of Phe-$R_1$-Lys-Pro-Phe-$R_2$-Phe-$R_3$-Met-$R_4$-$R_5$-$R_5$-$R_7$-$R_5$-Lys therein. In this sequence, $R_1$ through $R_7$ are as defined above and reflect possible amino acid substitutions in the core sequence, while $R_8$ is selected from the group consisting of Ser and Thr. Preferably this factor contains substantially only one peptide.

Within this first class, a more clearly defined factor comprises substantially only peptides including a sequence of Phe-Asn-Lys-Pro-Phe-Val-Phe-Leu-Met-Ile-Asp-Gln-Asn-Thr-Lys. This is the original sequence of the breast fragment. These peptides can include additional flanking sequences on either side of the specified sequence, and these flanking sequences can be of indeterminate length. Preferably this factor also contains substantially only one peptide.

The most closely defined factor within this first class comprises a substantially pure peptide consisting essentially of the amino acid sequence Phe-Asn-Lys-Pro-Phe-Val-Phe-Leu-Met-Ile-Asp-Gln-Asn-Thr-Lys. This is the exact sequence of the breast fragment with no flanking sequences.

The second class of these factors is derived from the sequence of the lung fragment, Phe-Asn-Lys-Pro-Phe-Val-Phe-Leu-Met-Ile-Asp-Gln-Asn-Phe-Ser-Lys. This class most broadly comprises substantially only peptides having at least sixteen amino acid residues including a sequence of Phe-$R_1$-Lys-Pro-Phe-$R_2$-Phe-$R_3$-Met-$R_4$-$R_5$-$R_5$-$R_7$-Phe-$R_8$-Lys therein. In this sequence, $R_1$ through $R_7$ are as defined above and reflect possible amino acid substitutions in the core sequence, while $R_8$ is selected from the group consisting of Ser and Thr. Preferably this factor contains substantially only one peptide.

Within this second class, a more clearly defined factor comprises substantially only peptides including a sequence of Phe-Asn-Lys-Pro-Phe-Val-Phe-Leu-Met-Ile-Asp-Gln-Asn-Phe-Ser-Lys. This is the original sequence of the lung fragment. These peptides can include additional flanking sequences on either side of the specified sequence, and these flanking sequences can be of indeterminate length. Preferably this factor also contains substantially only one peptide.

The most closely defined factor within this second class comprises a substantially pure peptide consisting essentially of the amino acid sequence Phe-Asn-Lys-Pro-Phe-Val-Phe-Leu-Met-Ile-Asp-Gln-Asn-Phe-Ser-Lys. This is the exact sequence of the lung fragment with no flanking sequences.

All of these factors are expected to have SCM activity and are within the scope of the present invention.

## 5. Cross-Reactivity of the SCM Factor

The factor is designated as a general cancer-associated SCM factor because lymphocytes isolated from donors with all types of cancer respond to the factor in the SCM test. The type of cancer afflicting the donor of the lymphocytes need not be the same as the type of cancer afflicting the donor of the body fluid from which the SCM factor was purified. As will be discussed below, purification of the factor from blood plasma obtained from patients with nineteen different types of cancer always resulted in a factor eluting in the same position in reverse phase high-pressure liquid chromatography, strongly suggesting that the same molecule or a very similar molecule was isolated from all these patients. In fact, amino acid sequencing of a tryptic fragment from the SCM factors isolated from blood plasma of patients with lung cancer and breast cancer shows that the first thirteen amino acids of the fragments are identical. The only difference between the two sequences is the deletion of a phenylalanine residue present in position 14 of the lung fragment and the conservative substitution of threonine for serine in the next position. Details on the demonstration of the cross-reactivity of the SCM factor are given hereinbelow, under "Examples."

6. Modification of the SCM Response by the General Cancer-Associated SCM Factor

The general cancer-associated SCM factor has the property of being able to modify the response of potentially SCM-responding lymphocytes obtained from donors free of malignancy when those lymphocytes are contacted with the factor. Before contact, the lymphocytes respond only to mitogens in the SCM test and do not respond to cancer-associated antigens. However, after prolonged contact with the SCM factor, the SCM response of the cells is modified to respond only to cancer-associated antigens and not to mitogens. In other words, contact by such lymphocytes with the SCM factor alters their response in the SCM test from the normal response of lymphocytes from donors free of malignancy to the response seen with lymphocytes from donors afflicted with cancer. Details on the demonstration of this modification of the SCM response of lymphocytes from donors free of malignancy are given hereinbelow under "Examples."

7. Effect of General Cancer-Associated SCM Factor on Natural Cytotoxicity of Lymphocytes

The SCM factor has the property of greatly decreasing the in vitro spontaneous natural cytotoxicity of potentially SCM-responding lymphocytes. On the basis of this property it is believed that the general cancer-associated SCM recognition factor molecules are involved in the defense of cancer cells against the attack of killer lymphocytes and in this way help the survival and unrestrained growth of cancer cells. The importance of the normal functioning of the immune system in controlling the growth of cancer cells is shown by the frequent occurrence of unusual forms of cancer in patients undergoing immune suppression. An important example of this is the occurrence of aggressive forms of Kaposi's sarcoma, ordinarily a rather slowly-spreading cancer, in patients afflicted with acquired immunodeficiency syndrome (AIDS).

Since the general cancer-associated SCM factor modifies the SCM responses of lymphocytes from healthy donors and decreases the natural cytotoxicity of killer lymphocytes toward myeloid cells, it is believed that treatment of a body fluid containing the SCM-recognition factor to reduce the in vivo effect of the factor by selectively inactivating it, followed by return of the body fluid to the patient, can be used in management of cancer in order to increase the natural resistance to the disease. When the body fluid is peripheral blood, the step of treating the body fluid can comprise the dialysis of the peripheral blood to remove peptides with an apparent molecular weight of less than 1000 daltons. Alternatively, the step of treating the body fluid can comprise neutralizing the factor with an antibody specific for it, or with fragments of such an antibody, such as Fab fragments. Such Fab fragments are capable of univalent binding so that each fragment molecule only binds one molecule of factor. This could be preferable to the use of intact antibody in some applications if the formation of large factor-antibody complexes is considered undesirable. This use of the general cancer-associated SCM cancer-recognition factor is described more fully hereinbelow.

Purification of the SCM Factor

The first step in purification of the SCM factor is obtaining an ultrafiltrate from a body fluid of a donor afflicted with cancer. The body fluid can be peripheral blood, blood plasma, or urine; if the fluid is peripheral blood, the blood must be centrifuged to separate the red blood cells from the plasma. The donor of the body fluid used for isolation of the SCM factor can be either autologous or allogeneic with respect to the lymphocytes used for the SCM test.

The ultrafiltration process separates a first fraction of the body fluid comprising molecules having an apparent molecular weight greater than 1000 daltons from a second fraction comprising molecules having an apparent molecular weight less than 1000 daltons. The general cancer-associated SCM factor of the present invention is found in the second fraction of the ultrafiltrate. The terms "apparent molecular weight" and "nominal molecular weight cutoff" are used herein because ultrafiltration is a somewhat imprecise method of separating molecules according to molecular weight in this molecular weight range, and the exact molecular weight excluded by a filter with a nominal molecular weight cutoff of 1000 daltons depends somewhat on the conformation of the molecule. Molecules larger than 1000 daltons in actual molecular weight can in fact pass through a filter with a nominal molecular weight cutoff of 1000 daltons if, for example, the molecules are relatively long and narrow.

Preferably, the separation of the second fraction from the first fraction is performed by filtration of the body fluid through an ultrafilter with a nominal 1000-dalton molecular weight cutoff.

The purity of a preparation of such a factor, at the ultrafiltrate stage or later, can be described by its specific activity. In this context, the term "specific activity" is defined as the reciprocal of the quantity of protein required to cause a particular degree of decrease, such as 20%, in the intracellular fluorescence polarization value when a particular fraction is used to challenge SCM-responding lymphocytes in the SCM test. The goal of purification of the SCM factor is to increase the specific activity of the SCM factor over the specific activity found in the crude ultrafiltrate. The process of purification can therefore be followed by determining the specific activity of the purified fractions. Since the protein concentration in the examples reported herein is only determined approximately in terms of ultraviolet absorbance, preferably at 220 nm, and the dose-response curve for the factor has not yet been determined, the characterization of various steps of the purification of the SCM factor described herein in terms of specific activity is only approximate. However, it is clear that the protein concentration decreases markedly as the factor moves through the various purification steps, while the activity is relatively unaffected, thereby resulting in an increase in specific activity of the SCM factor. Nevertheless, the ultrafiltrate can properly be described as consisting essentially of substantially pure general cancer-associated SCM-recognition factor, inasmuch as ultrafiltration through a membrane with a nominal molecular weight cutoff of 1000 will remove from a biological fluid the overwhelming majority of molecules with specific biological activity, including all proteins and larger peptides.

The next stage in the purification of the general cancer-associated SCM factor is a desalting step in which the second fraction obtained from ultrafiltration is loaded on a chromatographic column capable of separating the salts therefrom. The material loaded onto the column is then eluted from the column with distilled water, and the portion eluting at an elution volume between about 0.3 and about 0.5 times the total chromatographic bed volume, containing the SCM factor, is collected. Preferably the column used in this step is a gel filtration column with a fractionation range of from 0 to about 700 daltons, such as Sephadex$_{(TM)}$ G-10.

The next stage in the purification is another gel filtration step, again separating according to size. The SCM-containing material obtained from the desalting step is loaded onto another gel filtration column, with a fractionation range of from about 1500 daltons to about 30,000 daltons, such as Sephadex$_{(TM)}$ G-50. The material loaded onto the column is then eluted therefrom with a weak aqueous solution of an ammonium salt. Preferably the ammonium salt is ammonium bicarbonate, more preferably 50 mM ammonium bicarbonate. That portion eluting at an elution volume between about 0.4 times and about 0.6 times the total chromatographic bed volume contains the SCM factor and is collected.

The next stage in the purification is an anion-exchange chromatography step, separating by charge. The SCM factor-containing material from the previous gel filtration step is loaded onto an anion-exchange column, preferably diethylaminoethyl cellulose (DEAE cellulose). The material loaded onto the column is then eluted from the column with an increasing concentration of an ammonium salt. Preferably that ammonium salt is ammonium bicarbonate and the increasing concentration of the ammonium salt is from 10 mM to 1.0 M ammonium bicarbonate. The fraction eluting from the column at about 0.28 M to about 0.31 M ammonium bicarbonate contains the SCM factor and is collected.

The final stage of purification is reverse phase high pressure liquid chromatography (RP-HPLC), which separates by charge and/or hydrophobicity. Typically, the SCM factor-containing material from the DEAE-cellulose column eluate is loaded onto a Aquapore RP-300 RP-HPLC column with dimensions of 220 mm x 2.1 mm. Elution is then performed with a combination of two solvents, initially 90 volume percent of 0.1 volume percent aqueous trifluoroacetic acid (TFA) and 10 volume percent of 0.09% volume percent of TFA in aqueous 70 percent acetonitrile, followed by a gradient with an increasing concentration of the acetonitrile-containing solution. The SCM factor from all starting materials elutes as a homogeneous peak at a solvent composition of 26 volume percent of 0.1 volume percent aqueous trifluoroacetic acid and 74 volume percent of 0.09 volume percent aqueous trifluoroacetic acid in aqueous 70% acetonitrile.

Alternatively, RP-HPLC can be performed on a Beckman Ultrasphere ODS$_{(TM)}$, reverse phase high pressure liquid chromatography column. With this column, elution is then performed with a somewhat different solvent combination, initially 70 volume percent of 0.1 volume percent aqueous trifluoroacetic acid and 30 volume percent of 0.1 volume percent aqueous trifluoroacetic acid in aqueous 70% acetonitrile, followed by a gradient with an increasing concentration of the acetonitrile-containing solvent. The SCM factor always elutes as an homogeneous peak at a solvent composition of 43.7 volume percent of 0.1% aqueous trifluoroacetic acid and 56.3 volume percent of 0.1% aqueous trifluoroacetic acid in aqueous 70% acetonitrile when this column and this solvent system is used.

Use of the SCM Factor

## 1. Use in the SCM Test

The activity of the SCM factor is confirmed by its effect on viable potentially SCM-responding lymphocytes in accordance with the general test method described in the prior publication by L. Cercek and B. Cercek, "Application of the Phenomenon of Changes in the Structuredness of Cytoplasmic Matrix (SCM) in the Diagnosis of Malignant Disorders: a Review," Europ. J. Cancer 13, 903-915 (1977). The general cance-associated SCM-recognition factor of the present invention produces a significant decrease in the intracellular fluorescence polarization value of potentially SCM-responding lymphocytes from donors afflicted with cancer when used to challenge such lymphocytes in the SCM test performed as described in that article. The degree of decrease of the intracellular fluorescence polarization value of such challenged lymphocytes is substantial--at least 20% even if ultrafiltrate is used to challenge such lymphocytes, and more than 50% if the most purified HPLC fraction is used.

## a. Procedures for Carrying Out the SCM Test

Two previously established procedures are important for the proper performance of the SCM test as reported herein. These procedures are the isolation of potentially SCM-responding lymphocytes and the technique of measuring the fluorescence polarization values themselves, and their conversion into numbers meaningful for the SCM test. These procedures are summarized in the following discussion.

## (1) Isolation of Potentially SCM-Responding Lymphocytes

The isolation of potentially SCM-responding lymphocytes is described in the European Journal of Cancer review article, supra, and also in a prior patent application by B. Cercek & L. Cercek, Serial No. 838,264, filed March 10, 1986, entitled "Automated Collection of Buoyant Density Specific Cells from Density Gradients," and incorporated herein by this reference. The separation of these lymphocytes from the general lymphocyte population is important for the proper performance of the SCM test, because only a relatively small fraction of lymphocytes, approximately 20 to 25 percent, is capable of responding in the SCM test. Therefore, to perform the test on unfractionated lymphocytes results in a much smaller observed decrease In the intracellular polarization value even when the lymphocytes actually capable of responding in the SCM test fully responded.

For the isolation of potentially SCM-responding lymphocytes, a sample of peripheral blood is drawn from a donor and collected in a heparinized tube. After collection the peripheral blood is treated with iron powder or carbonyl-iron powder and the tubes containing the blood-iron powder mixture are placed on a magnet to effect separation of the phagocytic cells along with the iron powder from the blood sample. A portion of the blood sample is then transferred to a Ficoll $_{(TM)}$-Triosil$_{(TM)}$ density gradient solution and centrifuged to effect separation of the potentially SCM-responding lymphocytes based on density differences. This method of separation uses a density gradient solution having a density of 1.081 g/cm$^3$ at 25° C and an osmolality of 0.320 Osm/kg. Centrifugation is carried out at 550 x g for 20 min. at a temperature of 25° C. The potentially SCM-responding lymphocytes are recovered using a Pasteur pipette to remove the cell layer separated above the density gradient material. Care must be taken to avoid, as much as possible, the removal of the density gradient material as this material includes various heavier plasma and cell components which interfere with the test results. Removal of the lighter plasma materials should also be avoided as much as possible to eliminate the introduction of any contaminants or SCM-nonresponding cells into the test samples.

Following separation, the potentially SCM-responding lymphocytes are subjected to several washing steps, first in 0.9% preservative-free sodium chloride solution, then in complete Dulbecco's phosphate buffered saline (PBS) and held at 37° C for subsequent use in the SCM test procedure.

## (2) Measurement of SCM Values

The method for measuring the fluorescence polarization values of SCM-responding lymphocytes in the SCM test has been described in the European Journal of Cancer review article, supra, as well as in a prior patent application by B. Cercek & L. Cercek, Serial No. 867,079, filed May 27, 1986, entitled "Method for Measuring Polarized Fluorescence Emissions," incorporated herein by this reference. As described in these references, SCM-responding lymphocytes previously separated from the test subject's peripheral blood are incubated in sterile glass tubes at 37° C with a known concentration of either a mitogen such as phytohaemagglutinin or a cancer-associated antigen such as the general cancer-associated SCM-recognition factor which is the subject of the present invention. Other mitogens than phytohaemagglutinin (PHA), such as concanavalin A and pokeweed mitogen, have been used, but PHA is preferred for the SCM test. This incubation is initiated by adding 0.1 ml of the appropriately diluted mitogen or antigen to 1 ml of the cell suspension at 6 x $10^6$ cells/ml. The incubation is then allowed to proceed for 30-60 min.

The incubated lymphocytes are then admixed in suspension with a suitable nonfluorogenic compound hydrolyzable intracellularly to a fluorogenic compound, referred to hereinafter as a fluorogenic agent precursor, such as fluorescein diacetate (FDA). The fluorescein diacetate is used at a final concentration of 2.5 mM in complete PBS at pH 7.4 and osmolality of 0.330 Osm/kg and is diluted from a concentrated stock solution prepared in acetone or glacial acetic acid. Aliquots of 0.2 ml of control or stimulated lymphocyte suspensions are slowly injected with a syringe into a beaker containing 3 ml of the FDA substrate solution.

The cells are exposed to the FDA for sufficient time (about 5 minutes) to allow for the penetration of the FDA substrate solution into the lymphocytes. Inside the cells, the nonfluorogenic fluorescein diacetate molecules are converted to fluorescein molecules by enzymatic hydrolysis.

The fluor-containing lymphocytes are isotropic in their response to polarized light since the polarization of the emitted fluorescence relative to that of the exciting light does not depend on the orientation of the plane-polarized light used to excite the lymphocytes. However, the conventional fluorescence polarization measuring apparatus used herein for these measurements uses vertically polarized exciting light to excite the lymphocytes, so the measurement process is described in terms of vertically polarized exciting light.

When exposed to excitation energy in the form of vertically polarized light, the fluorescein molecules emit fluorescence. The relationship between the vertically polarized and horizontally polarized emissions is measured. This can be done by measuring the polarized fluorescence intensities in both the vertical and horizontal planes and determining a polarization value (P value) in accordance with the following relationship:

$$P = \frac{I_V - GI_H}{I_V + GI_H}$$

here $I_V$ and $I_H$, are polarized fluorescence intensities in the vertical and horizontal planes, respectively, and G is a correction factor for the unequal transmission of the horizontal and vertical components of the polarized light through the optical system of the particular equipment used. The value of G is determined by dividing the intensity of the horizontally polarized light by the intensity of the vertically polarized light emitted from a $10^{-7}$ M solution of fluorescein in PBS excited with horizontally polarized light of the same wavelength as used for the SCM measurements. For the measurements reported herein, G = 0.42.

The P value of stimulated lymphocytes, that is those lymphocytes that have been exposed to the general SCM-associated cancer recognition factor of the present invention, is compared with the P value of a control suspension of unstimulated lymphocytes from the same donor and the percent decrease in P value of the stimulated lymphocytes as compared to the P value of the control lymphocytes is an indication of the SCM-response to the cancer antigen.

Although the SCM response can be observed through some range of excitation and emission wavelengths, when using FDA as the fluorogenic agent precursor, it is strongly preferred to use an excitation wavelength of 470 nm and an emission wavelength of 510 nm. All results hereinafter described were obtained using those wavelengths. However, good results have also been achieved using an excitation wavelength of 442 nm and an emission wavelength of 527 nm.

The spectrophotometer utilized for SCM fluorescence measurements should be one of high sensitivity and stability and should be able to compensate for fluctuations in the intensity of the exciting light since the intensity of the polarized fluorescence emissions is recorded as a function of time and since the bulk concentration of fluorescein in the SCM measurements is only of the order of $10^{-8}$ M to $10^{-9}$ M. Also, broad band filter instruments are not suited for use for SCM measurements since SCM responses can be detected only within a narrow wavelength region. The maximum spectral slit width of the excitation monochromator should be 20 nm and the maximum spectral slit width of the emission monochromator should be 10 nm when the excitation monochromator is set at 470 nm and the emission monochromator at 510 nm. The spectrophotometer should also be fitted with a thermostatically controlled cuvette holder since the polarized fluorescent emissions are highly temperature dependent. The spectrophotometer should also be provided with means for measuring both the horizontal and vertical polarized components of the fluorescent emissions.

In the examples hereinafter set forth we used a Perkin-Elmer MPF-4 spectrophotometer which was equipped with a thermostatically controlled cuvette holder. All measurements were carried out at 27° C. The light source was a xenon lamp.

In measuring the fluorescence polarization, the intensities of the emissions parallel to and perpendicular to the vertical exciting light beam are recorded alternately with an automatic polarizer changer for about 6 minutes or until the intensity of the emission perpendicular to the vertically exciting light beam reaches 80-90% of the full scale deflection of the recorder.

It is necessary to correct these readings for any leakage of fluorescein from the cells and for any background of fluorescence in the substrate solution. To perform this correction, the cells are filtered away from the solution on a nitrocellulose filter of 0.22 μm pore size mounted in an appropriate filter head. Using the same fluorescence polarization measurement apparatus, the fluorescence intensities parallel to and perpendicular to the exciting light are obtained. The corrected fluoreScein intensities for the cells are then obtained by subtracting the values obtained from the filtrate from the total fluorescence intensities extrapolated to the half time of filtration. This extrapolation is necessary because the background increases during the incubation because of the leakage of fluorescein from cells and spontaneous hydrolysis of FDA.

Alternatively, the method of compensating for background fluorescence described in the prior patent application by the Cerceks, Serial No. 867,079, filed May 27, 1986, entitled "Method for Measuring Polarized Fluorescence Emissions," and incorporated herein by reference, can be used. Briefly, this method eliminates the need to filter each sample by measuring the horizontally and vertically polarized fluorescence emissions at more than one wavelength and calculating the intracellular fluorescence emissions therefrom.

An SCM test performed according to the protocol described hereinabove, using 1.0 ml of a lymphocyte suspension at $6 \times 10^6$ cells/ml and 0.1 ml of the mitogen or antigen, with FDA as the fluorogenic agent precursor and using an excitation wavelength of 470 nm and an emission wavelength of 510 nm, is referred to herein as a "standard SCM test."

b. Use of the General Cancer-Associated SCM Factor as a challenging Agent in the SCM Test

The general cancer-associated SCM factor whose purification is herein described can be used as an effective challenging agent in the SCM test for the detection of cancer. Thus cells from donors not afflicted with cancer exhibit no significant change in SCM response when stimulated by cancer antigens used as challenging agents in the SCM test, while cells from donors afflicted with cancer exhibit a significant change in SCM response. This change is seen as a decrease of the P value when cells from donors afflicted with cancer are stimulated with cancer-associated antigens. As described, the SCM factor is nonspecific with respect to the type of cancer detected; the lymphocytes challenged with the SCM factor in the SCM test need not have come from a donor with the same type of cancer as the donor of the body fluid from which the SCM factor came.

When used as a challenging agent in the SCM test, the SCM factor produces a decrease of P value of at least ten percent, and can produce a decrease of P value of as much as 44.6%. If $P_S$ is the P value for an aliquot of the challenged lymphocytes, and $P_C$ is the P value for an aliquot of the lymphocytes not challenged with SCM factor, a ratio of $P_S$ to $P_C$ of less than about 0.9 is an indication of the presence of malignancy in the body of the donor of the challenged lymphocytes.

A preferred method of using the SCM factor as a challenging agent in the SCM test comprises comparing $P_S$ to the fluorescence polarization value, $P_M$, of another aliquot of the lymphocytes contacted with a mitogen, to determine an SCM response ratio, $RR_{SCM}$, where:

$$RR_{SCM} = P_S / P_M.$$

An $RR_{SCM}$ of less than about 0.9 indicates the presence of a malignancy in the donor of the lymphocytes.

2. Use in the Management of Cancer

Since it is believed that the general cancer-associated SCM-recognition factor is involved in the defense of cancer cells against the in vivo attack of killer lymphocytes, it follows that measures that selectively decrease the in vivo activity of the SCM factor can be useful in the management of cancer by increasing the susceptibility of cancer cells to attack by killer lymphocytes. By "in vivo activity" is meant the ability of the SCM-recognition factor to decrease the cytotoxicity of killer lymphocytes toward malignant cells.

As a first step to such methods, once lymphocytes from a known or suspected cancer patient have been shown to give a positive response in the SCM test with the general cancer-associated SCM-recognition factor as the challenging agent, a sample of a body fluid can be taken from the patient, passed through a filter with a nominal molecular weight cutoff of 1000 daltons, and the fraction passing through the filter collected and used to challenge lymphocytes from the same patient in the SCM test to confirm the presence of cancer-associated SCM-recognition factor in the fraction. It need not always be necessary to perform this test, particularly if other clinical indicators indicate the presence of cancer.

Once the presence of SCM factor in a body fluid of a cancer patient is shown or inferred, the body fluid can be treated by one of several methods to reduce the in vivo effect of the factor by selectively inactivating it, and the body fluid can then be returned to the patient, thereby enhancing the resistance of the patient to the malignancy. Since the general cancer-associated SCM factor causes a response in the SCM test regardless of the type of cancer afflicting the patient, it follows that reducing the in vivo effect of the SCM factor can enhance the resistance of the patient not only to the particular type of cancer originally diagnosed, but also to any other type of malignancy which might subsequently develop. This can prove significant when treating patients with drugs that have an immunosuppressant effect, or patients with an already compromised immune system due to conditions such as AIDS.

When the body fluid is peripheral blood, one method of reducing the in vivo activity of the SCM factor is to physically remove it by dialysis of the peripheral blood to remove peptides with an apparent molecular weight of 1000 daltons or less, since the factor will pass through ultrafilters with a nominal molecular weight cutoff of 1000 daltons.

Another method of reducing the in vivo activity of the SCM factor in a body fluid is to neutralize it or inactivate it with antibody specific for the factor. The SCM factor can be covalently coupled to a larger carrier molecule such as polylysine, and antibodies prepared to the conjugate, the SCM factor acting as a hapten. Once such antibodies are prepared, conventional methods can be used to isolate antibody-forming cells and produce monoclonal antibodies using cell fusion with hybridomas. Once antibody, whether polyclonal or monoclonal, is formed, it can be used to neutralize the SCM factor.

Alternatively, once the antibody is formed, it can be cleaved into univalent Fab fragments with the proteolytic enzyme papain. Each fragment molecule produced by such cleavage can bind only one molecule of the factor, in contrast to intact antibody, which has sites for binding two separate molecules of factor. Use of the fragments can be preferable to use of intact antibody in some applications if the formation of large factor-antibody complexes is considered undesirable. The presence of such large antigen-antibody complexes in the peripheral blood can possibly cause serum sickness and other allergic reactions.

### 3. Additional Uses of Antibodies to the SCM Factor

An additional method of using antibodies produced to the SCM factor in the management of cancer is the tagging of the antibody with a substance with anti-cancer activity, in order to direct the anti-cancer substance to the site of the cancer and thereby raise the effective concentration of the anti-cancer substance at the site of the cancer. This procedure can be especially advantageous when the anti-cancer substance is one that produces side effects when given in larger doses.

The anti-SCM antibody can also be used to determine the levels of the SCM factor in peripheral blood or other bodily fluids by techniques such as radioimmunoassays, fluorescence immunoassays, or enzyme-linked immunosorbent assays, or by precipitation assay techniques such as nephelometry or turbidimetry, assuming that the SCM factor is not a univalent antigen. Assuming that the SCM factor is physically associated in some way with tumor cells, antibodies labeled with imaging substances such as fluorescent dyes or radioactive isotopes can also be used to image cancer cells so that cancer cells could be detected in biopsies. Also, fluorescent-labeled antibody can be used for automated detection of cancer cells by flow cytometry.

### EXAMPLES

The following examples illustrate the SCM factor, its isolation and purification, and its method of use. It will be understood, however, that the following examples are by way of illustration only and not by limitation.

### Example 1-Initial Purification of the General Cancer-Associated SCM Factor

Blood samples from patients positively diagnosed as having active cancer, such as cancer of the breast, lung, colon, ovary, cervix, uterus, larynx, or skin (basal cell carcinoma and malignant melanoma) were collected into heparinized vials such as Vacutainer$_{(TM)}$, tubes. Twenty-milliliter portions of the blood samples were centrifuged at about 1200 x $g$ for approximately 40 min. The plasma above the sedimented blood cells was collected and filtered by pressure through a porous membrane filter such as an Amicon$_{(TM)}$, UM2 or YM2 filter, with a 1000-dalton molecular weight cutoff. These ultrafiltrates were lyophilized or stored at 4° until further purification.

### Example 2-Tests of Initially Purified SCM Factor

Aliquots of the ultrafiltrate from each sample of Example 1 were incubated with potentially SCM-responding lymphocytes obtained from the same donors and the lymphocytes checked for their SCM response in accordance with the SCM test procedure described above. In every case the ultrafiltrate caused the SCM-responding lymphocytes to respond characteristically with a decrease in P value, as they would have if they had been contacted with the cancerous tissue itself or with extracts of cancerous tissue (Table 2).

## TABLE 2

### SCM ACTIVITY OF ULTRAFILTRATES OF EXAMPLE 1

| Diagnosis of Lymphocyte Donor | Diagnosis of SCM Factor Donor | SCM Response: P Value as % of Control |
| --- | --- | --- |
| Malignant Melanoma | Malignant Melanoma | 75.7 |
| Malignant Melanoma | Basal Cell Carcinoma-Skin | 82.0 |
| Ca-Larynx | Ca-Larynx | 62.9 |
| Ca-Breast | Ca-Breast | 76.0 |

The data of Table 2 show that even when present in the crude ultrafiltrate, the SCM factor caused a decrease in the P value of the SCM-responding lymphocytes from donors afflicted with cancer at least equivalent to the decrease of the P values observed when lymphocytes are stimulated by crude extracts of cancerous tissues or cancerous tissues themselves. The decrease in P value on stimulation by the ultrafiltrates was at least 10%, which is characteristic of such a positive SCM response. However, the SCM factor did not pass through the Amicon(TM) UMO5 filter with a nominal 500-dalton molecular weight cutoff. These data confirm the small size of the factor while indicating that the activity is larger than a small molecule such as a single amino acid.

Example 3-Further Purification of the SCM Factor

The lyophilized powder from the samples of Example 1 was dissolved in 2 ml of sterile preservative-free water for injections. At this stage, the SCM activity of the preparations was ascertained, and active samples from donors with the same type and site of cancer were pooled. The pooled samples were desalted on an 0.9 x 18 cm column of Sephadex(TM) G-10, which has a fractionation range of from 0 to 700 daltons. The sample volume per column chromatographic run did not exceed 25% of the column volume. Elution was carried out with double distilled water at the linear elution speed of 8 to 9 cm/hr. The desalting was carried out at room temperature (21°-23° C). One-ml fractions eluting at between 0.3 and 0.5 times the total chromatographic bed volume were collected and the optical densities of the fractions determined. The SCM activity was contained within the first elution peak. The presence of SCM activity in that peak was confirmed by an SCM test. An aliquot of the first elution peak, prepared from an ultrafiltrate originally derived from plasma of a patient with breast cancer reduced the P value of lymphocytes from a patient with breast cancer to 86.3% of the control value in the SCM test, indicating the presence of SCM activity. These fractions were collected and lyophilized.

The eluate was further purified by $_n$ fractionation on a Sephadex(TM) G-50 gel filtration column, which has a fractionation range of from 1500 to 30,000 daltons. The lyophilized desalted samples were dissolved in 50 mM $NH_4 HCO_3$, loaded at no more than 5% of the column volume on a 0.9 x 18 cm Sephadex G-50 column at the linear elution speed of 3 cm/hr. The elution was carried out at room temperature, and one-milliliter fractions eluting from the column at between 0.4 and 0.6 times the total chromatographic bed volume were collected. These fractions were tested for SCM activity. Results of these tests are given below in Example 4. The SCM-active fractions were contained within the first elution peak as determined by optical densities of the one-milliliter fractions after testing of the fractions in the SCM test.

Once the fractions were tested for SCM activity, the active fractions from the same cancer types were pooled and lyophilized.

For further purification the lyophilized samples were dissolved in 10 mM $NH_4HCO_3$ and loaded at no more than 4% of the column volume on an 0.8 x 26 cm column of Whatman DE-52 microgranular DEAE-cellulose. The column was washed with 10 ml of 10 mM aqueous $NH_4HCO_3$ increasing by 0.108% per minute from 10 mM to 1 M $NH_4HCO_3$. One-milliliter fractions were collected and the optical absorption at 220 nm was determined for each fraction. Based on the optical absorbance, active fractions eluting from the column at between 4.5 and 4.7 times the total chromatographic bed volume were pooled and lyophilized for testing and further purification. Results from SCM testing of the active fractions are given in Example 4.

Example 4-SCM Tests of Further Purified Preparations

Table 3 shows the results when aliquots of the Sephadex G-50 fractions from Example 3 originally from donors with various types of cancer were used to challenge lymphocytes from donors, also with various types of cancer, in the SCM test. It can be seen that potentially SCM-responding lymphocytes have the same characteristic response to the G-50 fractions as they did to the previously characterized cancer-associated antigens. This desalted partially purified proteinaceous material exhibits a generally increased SCM response as compared to the crude ultrafiltrate for which the results were shown in Table 2. This increased SCM response is shown by decreased P values.

## TABLE 3

### SCM ACTIVITY OF SEPHADEX G-50 FRACTIONS OF EXAMPLE 3

| Diagnosis of Lymphocyte Donor | Diagnosis of SCM Factor Donor | SCM Response: P Value as % of Control |
|---|---|---|
| Ca-Lung | Ca-Breast | 73.0 |
| Ca-Lung | Ca-Cervix | 69.6 |
| Ca-Lung | Ca-Bronchus | 73.6 |
| Ca-Larynx | Ca-Bronchus | 77.6 |
| Ca-Breast | Ca-Bronchus | 80.2 |
| Ca-Colon | Ca-Breast | 63.5 |
| Ca-Larynx | Ca-Breast | 63.0 |
| Malignant Melanoma | Malignant Melanoma | 74.9 |
| Healthy Donor | Malignant Melanoma | 99.3 |
| Healthy Donor | Ca-Colon | 98.0 |
| Colitis | Ca-Colon | 98.9 |

Table 4 shows the results when SCM factor obtained from donors with various types of malignancies after purification through the DEAE-cellulose stage was used to challenge lymphocytes isolated either from donors with various types of malignancies or from donors free of malignancy in the SCM test. As expected, the lymphocytes from cancer patients responded to the SCM factors purified from the DEAE-cellulose columns with a considerable decrease in P value, while lymphocytes from donors free of malignant disease showed no such decrease in P value.

## TABLE 4

SCM ACTIVITY OF DEAE-CELLULOSE FRACTIONS
OF EXAMPLE 3

| Diagnosis of Lymphocyte Donor | Diagnosis of SCM Factor Donor | SCM Response: P Value as % of Control |
|---|---|---|
| Ca-Breast | Ca-Breast | 69.2 |
| Ca-Breast | Ca-Bronchus | 69.5 |
| Ca-Breast | Ca-Cervix | 69.0 |
| Basal Cell Carcinoma-Skin | Basal Cell Carcinoma-Skin | 82.0 |
| Healthy Donor | Ca-Colon | 98.6 |
| Cholecystitis | Malignant Melanoma | 100.0 |
| Urethritis | Ca-Cervix | 99.8 |
| Appendicitis | Ca-Colon | 98.0 |
| Benign Breast Growth | Ca-Breast | 97.8 |
| Benign Pituitary Adenoma | Ca-Brain | 100.0 |

Example 5-Final Purification of SCM Factor by RP-HPLC

The DE-52 general cancer-associated SCM-active fractions of Example 4 were then reconstituted and purified to homogeneity by reverse phase high pressure liquid chromatography (RP-HPLC) using a 2.1 mm x 22 cm HPLC column. The column was packed with Aquapore RP-300$_{(TM)}$ (7 microns). The mobile phases used in the RP-HPLC purification step were as follows:

Phase A: 0.1 volume percent aqueous trifluoroacetic acid (TFA).

Phase B: 0.09 volume percent aqueous TFA in aqueous 70% acetonitrile.

Lyophilized DE-52 SCM-active fractions were reconstituted with sterile water for injections (without preservatives) and 250 microliter aliquots were injected into the RP-HPLC column. The mobile phase flow rate was 50 microliters per minute and its composition profile was 10 minutes of 90 volume percent of Phase A, 10 volume percent of Phase B, followed by 30 minutes of linear increase of Phase B at the rate of 3 volume percent per minute. The optical density peaks detected by optical absorbance at 220 nm were hand-collected via a "nanobore" teflon tubing into 1.5 ml plastic conical Eppendorf centrifuge tubes and the solvent was evaporated in a vacuum centrifuge. In all cases, the general cancer-associated SCM-recognition factor eluted from the column at 74 volume percent of Phase B.

Example 6-Alternative RP-HPLC Purification of SCM Factor

Alternatively, the SCM factor can be purified by performing HPLC using a 4.6 mm x 25 cm HPLC column packed with Ultrasphere ODS$_{(TM)}$ (5 microns) distributed by Beckman Instruments, Inc. with the DEAE-52 SCM-active fractions of Example 4. The mobile phases used with this column were as follows:
   Phase A: 0.1 volume percent aqueous trifluoroacetic acid (TFA).
   Phase B: 0.1 volume percent TFA in aqueous 70% acetonitrile.
The same general procedure was followed with this column as for the Aquapore column, except that the mobile phase flow rate was 1.00 ml per minute and its composition profile was 5 minutes of 70 volume percent of Phase A, 30 volume percent of Phase B, followed by 20 minutes of linear increase of Phase B at the rate of 3.5 volume percent per minute. The optical density peaks were detected at 220 nm and were hand-collected into siliconized glass test tubes and the solvent was evaporated in a vacuum centrifuge. When this HPLC system was used, in all cases the purification of general cancer-associated SCM-recognition factor from nineteen different cancer types, including squamous cell carcinoma of the cervix, adenocarcinoma of the breast, adenocarcinoma of the bronchus, and malignant melanoma, always yielded a single optical density peak of activity, eluting at 56.3 volume percent of Phase B.

Example 7-SCM Tests of RP-HPLC Purified Preparations of Example 6

For SCM activity testing of the peptides isolated by RP-HPLC on the Ultrasphere column in Example 6, the peptides were reconstituted with sterile water for injections without preservatives. The SCM activity of SCM-responding lymphocytes after incubation with these samples is shown in Table 5. This fraction gives the greatest decrease in polarization value when used to challenge lymphocytes from donors afflicted with cancer. Two of the three preparations of SCM factor gave a decrease in polarization value greater than 40%, a larger decrease than seen with any other fraction tested. The purified factor, as expected, was non-specific with respect to the type of cancer afflicting the donor of the lymphocytes used. Also as expected, the purified factor gave no response when used to challenge lymphocytes from healthy donors.

## TABLE 5

### SCM ACTIVITY OF RP-HPLC FRACTIONS OF EXAMPLE 6

| Diagnosis of Lymphocyte Donor | Diagnosis of SCM Factor Donor | SCM Response: P Value as % of Control |
|---|---|---|
| Ca-Breast | Ca-Breast | 58.9 |
| Ca-Breast | Ca-Lung | 57.3 |
| Ca-Colon | Ca-Breast | 55.4 |
| Ca-Breast | Ca-Bronchus | 68.0 |
| Healthy Donor | Ca-Breast | 99.8 |
| Healthy Donor | Ca-Lung | 101.0 |

Example 8-Identification and Isolation of SCM-Active Tryptic Peptides from SCM Factor

A sixteen-amino-acid long tryptic fragment was isolated from the purified SCM factor of Example 5. This fragment was shown to have the full SCM activity of the larger peptide, and was sequenced by the automated Edman degradation procedure. The cleavage of the purified SCM factor with trypsin and purification of the active fragment was carried out by the following procedure:

To prevent adsorption loss of the peptide during lyophilization, the SCM factor was digested with trypsin in the presence of HPLC eluants. Trypsin digestion was carried out in 0.1 M Tris-HCl buffer, pH 8.3, at 37° for 24 hours using 10 percent by weight of trypsin. The digest was diluted fourfold with 0.1 volume-percent aqueous trifluoroacetic acid, and was injected into an Applied Biosystems 130A microflow HPLC-separation system. The tryptic fragments were separated using an Aquapore RP-300 column (200 mm x 2.1 mm). For the elution of the fragments, the mobile phase solvents were:

Phase A: 0.1 volume percent aqueous trifluoroacetic acid (TFA).

Phase B: 0.09 volume percent TFA in aqueous 70% acetonitrile.

The mobile phase flow rate was 50 µl per minute and the composition profile was 10 minutes of 96 volume percent Phase A, 4 volume percent Phase B, followed by a linear elution gradient comprising a 30 min linear increase in Phase B at a 3 volume percent per minute rate. The SCM-active tryptic peptide fragment eluted at 69.6 volume percent of Phase B and 30.4 volume percent of Phase A in a total volume of about 30 microliters.

Example 9-Use of the SCM-Factor as the Challenging Agent in the SCM Test

Table 6 summarizes the results obtained by using preparations of the general cancer-associated SCM recognition factor at various stages of purification from Examples 1, 3, and 6 as the challenging agent in the SCM test. When lymphocytes from donors afflicted with a number of different malignancies were used with the factor of the present invention in the SCM test, a significant response was seen in all cases. This response is given in Table 6 as a percent of the control polarization value obtained by performing the SCM measurement on the same lymphocytes unincubated with the factor. The smaller the value the greater the response to the factor in the SCM test. Even the crudest preparation of the factor tested, the ultrafiltrate, give a decrease in polarization value of from 18.0% to 37.1%, and the most highly purified fraction, purified by RP-HPLC, gave a decrease in polarization value of as great as 44.6%. The factor of the present invention is specific and only causes a decrease in polarization value when used to challenge lymphocytes from donors afflicted with cancer. Even the RP-HPLC purified fraction caused no decrease in polarization value when used to challenge lymphocytes from healthy donors.

## EXAMPLES OF SCM ACTIVITY AT DIFFERENT PURIFICATION STAGES
### (EXAMPLE 9)

| Stage of Purification | Range of Percentage Decrease in P Value When Lymphocytes from Cancer Patients Used |
|---|---|
| Ultrafiltrate of Plasma (1000-Dalton Cutoff) | 18.0-37.1 |
| Sephadex G-50 Fraction | 19.8-37.0 |
| DE-52 Cellulose Fraction | 18.0-31.0 |
| RP-HPLC Fraction | 32.0-44.6 |

No fraction showed any activity when tested against lymphocytes from healthy donors, or from donors with non-malignant diseases.

Example 10-Use of the SCM-Active Tryptic Peptide of Example 8 in the SCM Test

The tryptic peptide whose purification was described above in Example 8 is fully active in the standard SCM test. Approximately $5 \times 10^{-2}$ femtograms of this fragment (i.e., $5 \times 10^{-17}$ grams, or approximately 16,000 molecules of the fragment) gave full activity in the test. When the fragment was isolated from patients with lung cancer, it proved active in the SCM test when tested against lymphocytes from a patient with small cell lung carcinoma, and crossreacted fully with lymphocytes from a patient with adenocarcinoma of the breast, as shown in Table 7. However, no response was seen when lymphocytes from healthy donors were used.

## TABLE 7

### SCM ACTIVITY OF TRYPTIC FRAGMENT OF EXAMPLE 8 FROM LUNG CANCER

| Diagnosis of Lymphocyte Donor | SCM Response: P Value as Percent of Control |
|---|---|
| Small Cell Lung Carcinoma | 70.0; 68.0 |
| Adenocarcinoma of Breast | 67.5; 68.0 |
| Healthy Donors | 102.0; 104.0 |

Example 11-Cross-Reactivity of the SCM Factor

The following example demonstrates the ability of the SCM factor to cause a response in the SCM test when used to challenge lymphocytes derived from donors afflicted with dissimilar types of cancer. In order to demonstrate this cross-reactivity, two milliliters of cell-free blood plasma was obtained from each of a number of blood samples from cancer patients. The blood samples had originally been collected into heparinized Vacutainer(TM) tubes. The samples were ultrafiltered through an Amicon UM2 or YM2 filter with a nominal molecular weight cutoff of 1000 daltons for at least 12 hr and stored under sterile conditions at 4°C. Potentially SCM-responding lymphocytes were isolated from heparinized blood samples from patients with cancer, healthy donors, and donors with non-malignant diseases. To test the activity and cancer specificity of the general cancer-associated SCM-recognition factor containing ultrafiltrates, 0.75 ml aliquots of potentially SCM-responding lymphocytes ($5 \times 10^6$ cells/ml in PBS) were incubated for 40 min at 37°C with the ultrafiltrates. 0.075 ml of the ultrafiltrates was used for each assay. SCM measurements were carried out as previously described in the article by L. Cercek and B. Cercek, "Application of the Phenomenon of Changes in the Structuredness of Cytoplasmic Matrix (SCM) in the Diagnosis of Malignant Disorders: a Review," Europ. J. Cancer 13, 903-915 (1977) and in the prior patent application by B. Cercek and L. Cercek, Serial No. 867,079, filed May 27, 1986, and entitled "Method for Measuring Polarized Fluorescence Emissions." In the SCM assay, a decrease in the intracellular fluorescence polarization value (P value) of at least 10% was taken as a positive response to the challenging ultrafiltrate.

**TABLE 8**

CROSSREACTIVITY OF SCM ACTIVITY OF ULTRAFILTRATES OF EXAMPLE 11

| Diagnosis of Donor of Ultrafiltrate | Cases Responding/Cases Tested for Lymphocyte Donor with Diagnosis of: | | | | | | | | | |
|---|---|---|---|---|---|---|---|---|---|---|
| | Ca-Mouth | Ca-Larynx | Ca-Cervix | Ca-Ovary | Ca-Lung | Ca-Brain | Ca-Breast | Ca-Colon | Non-Cancer Diseases | Healthy Donors |
| Ca-Lung | -- | 2/2 | 3/3 | -- | 5/5 | 3/3 | 3/3 | 2/2 | 0/3 | 0/3 |
| Ca-Bronchus | 1/1 | 1/1 | 2/2 | 2/2 | 2/2 | -- | 2/2 | 1/1 | -- | 0/2 |
| Ca-Breast | 1/1 | 1/1 | 3/3 | 2/2 | 2/2 | -- | 3/3 | 1/1 | 0/3 | 0/2 |
| Ca-Ovary | 1/1 | 2/2 | 2/2 | 3/3 | 2/2 | -- | 2/2 | -- | 0/4 | 0/2 |
| Ca-Cervix | 1/1 | 1/1 | 5/5 | 1/1 | 2/2 | -- | 2/2 | 2/2 | 0/3 | 0/3 |
| Ca-Colon | 2/2 | -- | 3/3 | -- | 2/2 | -- | 2/2 | 1/1 | 0/2 | 0/2 |
| Ca-Mouth | 2/2 | -- | 1/1 | -- | 1/1 | -- | 1/1 | 1/1 | -- | 0/2 |
| Ca-Larynx | 1/1 | 6/6 | 3/3 | -- | 2/2 | -- | 2/2 | 1/1 | 0/2 | -- |
| Malignant Melanoma | -- | -- | -- | -- | -- | -- | 1/1 | 1/1 | -- | 0/2 |
| Glioblastoma | -- | -- | 2/2 | -- | -- | 2/2 | 2/2 | 1/1 | 0/2 | 0/2 |
| Appendicitis | -- | -- | -- | -- | 0/1 | -- | 0/1 | 0/3 | 0/3 | 0/2 |
| Urethritis | -- | -- | 0/2 | -- | 0/2 | -- | 0/1 | 0/2 | 0/3 | 0/3 |
| Infectious Abscess | -- | 0/1 | 0/1 | -- | -- | -- | 0/1 | 0/1 | 0/2 | 0/2 |
| Colitis | -- | -- | 0/1 | -- | -- | -- | 0/1 | 0/3 | 0/2 | 0/2 |
| Benign Pituitary Adenoma | -- | -- | -- | -- | 0/2 | 0/2 | 0/2 | -- | 0/1 | 0/1 |
| Healthy Donors | -- | -- | 0/2 | 0/1 | 0/3 | -- | 0/3 | 0/1 | 0/4 | 0/6 |

As the data of Table 8 show, potentially SCM-responding lymphocytes from patients with eight different cancer types responded to ultrafiltrates containing general cancer-associated SCM-recognition factors from nine different cancer types. In contrast, ultrafiltrates from plasmas of healthy donors and donors with non-cancer diseases did not trigger any positive SCM responses. Also, neither did the potentially SCM-responding lymphocytes from healthy donors or those with non-malignant conditions respond to any of the ultrafiltrates in the SCM test.

Example 12-Modification of the SCM Response by the SCM Factor

To demonstrate the modification of the SCM response of lymphocytes free of malignancy by incubation with the SCM factor, potentially SCM-responding lymphocytes were isolated from the blood samples of healthy donors and suspended in complete Dulbecco's phosphate buffered saline (PBS) at $5 \times 10^5$ cells/ml as described in the European Journal of Cancer article, supra, and also in the prior patent application by B. Cercek, Serial No. 838,264, filed March 10, 1986 and entitled "Automated Collection of Buoyant Density Specific Cells from Density Gradients." Aliquots of these cells were incubated in 3 ml of the following: (a) cell-free blood plasma from cancer patients; (b) plasma from cancer patients ultrafiltered for 12 hours through an Amicon UM2 filter with a molecular weight cutoff of 1000 daltons; (c) plasma from cancer patients ultrafiltered for 12 hours through an Amicon UM5 filter with a nominal molecular weight cutoff of 500 daltons; (d) general cancer-associated SCM factor as purified through the desalting or Sephadex G-10 column stage; (e) the factor as purified through the Sephadex G-50 column stage; (f) the factor as purified through the DEAE-cellulose column step; (g) the factor as finally purified through the RP-HPLC step; and (h) plasma from healthy donors ultrafiltered through an Amicon UM2 filter with a nominal molecular weight cutoff of 1000 daltons. These incubations were performed for 2.5 hr.

The ability of the SCM factor to modify the SCM response of the lymphocytes from healthy donors was demonstrated by determining the SCM response ratio ($RR_{SCM}$) of the lymphocytes before and after the incubation with each of the fractions described above. Before being contacted with either a mitogen or with the SCM factor for determination of the $RR_{SCM}$ the incubated cells were thoroughly washed. The presence or absence of modification was determined by the ratio of the polarization value of the lymphocyte suspension after a short contact period with substrates containing the SCM factor over the polarization value of the lymphocyte suspension after a short contact period with phytohaemagglutinin (PHA). In accordance with the SCM test procedure, a $RR_{SCM}$ of less than 1.0 is a positive indication of the presence of malignancy in the donor while an $RR_{SCM}$ of 1.1 or greater indicates the absence of malignancy.

## TABLE 9

### MODULATION OF SCM RESPONSES OF LYMPHOCYTES FROM HEALTHY DONORS BY SCM FACTOR (EXAMPLE 12)

| SCM Preparation Used for Modulation | $RR_{SCM}$: Before Modulation | After Modulation |
|---|---|---|
| Cell-free Cancer Blood Plasma | 1.38 | 0.80 |
| Ultrafiltrate of Cancer Blood Plasma (1000 Daltons Cutoff) | 1.35 | 0.78 |
| Sephadex G-10 (SCM-Active) | 1.40 | 0.80 |
| Sephadex G-50 (SCM-Active) | 1.38 | 0.73 |
| DEAE-Cellulose (SCM-Active) | 1.39 | 0.70 |
| RP-HPLC (SCM-Active) | 1.40 | 0.65 |
| Ultrafiltrate of Cancer Blood Plasma (500 Daltons Cutoff) | 1.38 | 1.38 |
| Ultrafiltrate from Autologous and Allogeneic Blood Plasma from Healthy Donors | 1.38 | 1.38 |

Table 9 indicates the effect of the incubation with SCM-factor-containing fractions on the response of the lymphocytes to either SCM factor or phytohaemagglutinin, as reflected in the $RR_{SCM}$. Lymphocytes which were either not preincubated, or which were preincubated with ultrafiltrate from healthy donors filtered through a filter with a nominal 1000-dalton molecular weight cutoff, or which were preincubated with ultrafiltrate from donors with cancer filtered through a filter with a nominal 500-dalton molecular weight cutoff, showed an $RR_{SCM}$ of 1.35 or higher, as expected. By contrast, lymphocytes which were preincubated with fractions containing SCM factor all showed decreases in the $RR_{SCM}$ to a value of 0.65-0.80 characteristic of lymphocytes originally isolated from patients with malignant disease.

Example 13-Effect of the SCM Factor on Lymphocyte Cytotoxicity

To demonstrate the effect of the SCM factor on the natural cytotoxicity of potentially SCM-responding lymphocytes toward malignant cells, such lymphocytes obtained from healthy donors were incubated for 2 1/2 hr at 37°C with plasma containing SCM factor isolated as previously described from blood samples of donors afflicted with cancer. Aliquots of these lymphocytes were also retained as controls and not incubated. In addition, potentially SCM-resgonding lymphocytes were obtained from donors having cancer, and treated in the same manner--some aliquots incubated with plasma containing SCM factor and others retained as controls and not incubated.

After incubation the cytotoxicity of the lymphocytes was tested in accordance with the method described in M.R. Potter and M. Moore, "Natural Cytotoxic Reactivity of Human Lymphocyte Subpopulations," Immunology 37, 187-194 (1979). In accordance with this published method cells of the K 562 human myeloid cell line labeled with $^{51}$Cr were used as target cells for the assay. The potentially SCM-responding lymphocytes were used as the effector cells. The ratio of target cells to effector cells was 1 to 20. Release of the $^{51}$Cr indicates that the effector cells are toxic to the target cell. The percent of cytotoxicity is determined as follows:

$$\text{Percent cytotoxicity} = \frac{R_S - R_C}{R_T - R_C},$$

where $R_S$ is the percent of $^{51}$Cr release in the sample, $R_C$ the percent of $^{51}$Cr release in the control and $R_T$ is the percent of $^{51}$Cr release in the presence of a detergent, Triton X-100. The results are shown in Table 10. These results show that incubation of potentially SCM-responding lymphocytes from healthy donors for 2.5 hr with ultrafiltrates filtered through filter with a nominal 1000-dalton molecular weight cutoff decreased their cytotoxicity by over 90%. When the incubation was performed with potentially SCM-responding lymphocytes from cancer patients, the decrease in cytotoxicity was smaller, between 40 and 90%. However, such lymphocytes from cancer patients had lower levels of cytotoxicity before incubation, and the residual level of cytotoxicity remaining after incubation with ultrafiltrate was comparable to that remaining after incubation of lymphocytes from healthy donors. The lower level of cytotoxicity present in cells from cancer patients was consistent with a decrease of such cytotoxicity caused by in vivo exposure to factors such as the general cancer-associated SCM recognition factor of the present invention.

TABLE 10

EFFECT OF SCM FACTOR ON NATURAL LYMPHOCYTE TOXICITY AGAINST K 562 HUMAN MYELOID CELL LINE
(EXAMPLE 13)

| Diagnosis of Donor of Potentially SCM-Responding Lymphocytes | Diagnosis of Donor of SCM Factor as Ultrafiltrate | Percent Cytotoxicity of Lymphocytes: | | Percent Decrease in Cytotoxicity |
|---|---|---|---|---|
| | | Before Incubation | After Incubation | |
| Healthy Donor #1 | Ca-Cervix | 40.0 | 2.2 | 94.5 |
| Healthy Donor #2 | Ca-Bronchus | 30.0 | 1.7 | 94.3 |
| Healthy Donor #3 | Ca-Larynx | 11.0 | 0.76 | 93.1 |
| Healthy Donor #4 | Ca-Larynx | 22.0 | 2.2 | 90.0 |
| Healthy Donor #5 | Ca-Pharynx | 41.0 | 0.33 | 99.2 |
| Ca-Tongue | Ca-Larynx | 23.0 | 2.1 | 90.9 |
| Ca-Lip | Ca-Bronchus | 7.4 | 2.7 | 63.5 |
| Ca-Ovary | Ca-Bronchus | 10.0 | 6.1 | 39.0 |
| Ca-Cervix | Ca-Cervix | 25.2 | 1.5 | 94.0 |
| Ca-Bronchus | Ca-Cervix | 29.6 | 3.1 | 89.5 |

Although the present invention has been described in considerable detail with regard to certain preferred versions thereof, other versions are possible Therefore the spirit and scope of the appended claims should not be limited to the description of the preferred versions contained herein.

**Claims**

1. A substantially pure general cancer-associated SCM-recognition factor consisting essentially of low molecular weight peptide passing through filters with a nominal 1000-dalton molecular weight cutoff and retained by filters with a nominal 500-dalton molecular weight cutoff and including the amino acid sequence Lys-Pro-Phe, the factor producing at least a ten percent decrease in the intracellular fluorescence polarization value of SCM-responding lymphocytes from donors afflicted with cancer as measured by the standard SCM test.

2. The factor of claim 1 purified to substantial homogeneity.

3. A general cancer-associated SCM-recognition factor comprising substantially only peptides having at least thirteen amino acid residues including a sequence of Phe-$R_1$-Lys-Pro-Phe-$R_2$-Phe-$R_3$-Met-$R_4$-$R_5$-$R_6$-$R_7$ therein, wherein $R_1$ is selected from Asn and Gln, $R_2$, $R_3$, and $R_4$ are each independently selected from Val, Leu, and Ile, $R_5$ is selected from Asp and Glu, and $R_6$ and $R_7$ are each independently selected from Asn and Gln.

4. The factor of claim 3 having the property of modifying the SCM response of SCM-responding lymphocytes obtained from donors free of malignancy when the factor is contacted with such SCM-responding lymphocytes, whereby the lymphocytes respond in the SCM test to cancer-associated antigens but not to mitogens after the contact.

5. The factor of claim 3 or 4 having the property of decreasing the spontaneous in vitro toxicity of potentially SCM-responding lymphocytes toward the human myeloid cell line K 562 by at least 90 percent as measured

by $^{51}$Cr release.

6. The factor of any one of the preceding claims having the approximate amino acid composition of ($Asx_2$, $Glx_3$, Ser, His, $Gly_5$, Thr, Arg, $Ala_3$, Tyr, Met, $Val_3$, $Phe_3$, Ile, $Leu_3$, $Lys_2$).

7. The factor of any one of claims 1-5 wherein a sixteen-amino-acid segment within the factor has the amino acid sequence Phe-Asn-Lys-Pro-Phe-Val-Phe-Leu-Met-Ile-Asp-Gln-Asn-Phe-Ser-Lys, the segment not including the amino terminus of the factor.

8. The factor of any one of claims 1-5 wherein a fifteen-amino-acid segment within the factor has the amino acid sequence Phe-Asn-Lys-Pro-Phe-Val-Phe-Leu-Met-Ile-Asp-Gln-Asn-Thr-Lys, the segment not including the amino terminus of the factor.

9. The factor of any one of claims 1-5 comprising substantially only peptides having at least fifteen amino acid residues including a sequence of Phe-$R_1$-Lys-Pro-Phe-$R_2$-Phe-$R_3$-Met-$R_4$-$R_5$-$R_6$-$R_7$-$R_8$-Lys therein, wherein $R_8$ is selected from Ser and Thr.

10. The factor of claim 9 comprising substantially only peptides including a sequence of Phe-Asn-Lys-Pro-Phe-Val-Phe-Leu-Met-Ile-Asp-Gln-Asn-Thr-Lys therein.

11. The factor of any one of claims 1-5 comprising substantially only peptides having at least sixteen amino acid residues including a sequence of Phe-$R_1$-Lys-Pro-Phe-$R_2$-Phe-$R_3$-Met-$R_4$-$R_5$-$R_6$-$R_7$-Phe-$R_5$-Lys therein, wherein $R_8$ is selected from Ser and Thr.

12. The factor of claim 11 comprising substantially only peptides including a sequence of Phe-Asn-Lys-Pro-Phe-Val-Phe-Leu-Met-Ile-Asp-Gln-Asn-Phe-Ser-Lys therein.

13. The factor of any one of the preceding claims containing substantially only one peptide.

14. The peptide of claim 12 and 13 wherein $5 \times 10^2$ femtograms of the peptide produces a decrease in the polarization value of at least 30 percent when used to challenge SCM-responding lymphocytes from donors afflicted with cancer in the SCM test.

15. A method for testing lymphocytes obtained from a mammalian donor for the presence or absence of malignancy in the donor comprising the steps of:
   (a) contacting a suspension of the lymphocytes with the general cancer-associated SCM-recognition factor of any one of the preceding claims; and
   (b) determining the decrease in the structuredness of the cytoplasmic matrix of the lymphocytes resulting from the step of contacting the suspension of the lymphocytes.

16. The method of claim 15 wherein the step of determining the decrease in the structuredness of the cytoplasmic matrix comprises the steps of:
   (a) measuring the fluorescence polarization, $P_S$, for an aliquot of the lymphocytes that has been contacted with the factor;
   (b) measuring the fluorescence polarization, $P_C$, for a second aliquot of the lymphocytes that has not been contacted with the factor; and
   (c) determining the ratio of $P_S$ to $P_C$, whereby a ratio of $P_S$ to $P_C$ of less than about 0.9 indicates the presence of a malignancy in the donor of the lymphocytes.

17. The method of claim 15 wherein the step of determining the decrease in the structuredness of the cytoplasmic matrix comprises the steps of:
   (a) measuring the fluorescence polarization, $P_S$, for an aliquot of the lymphocytes that has been contacted with the factor;
   (b) measuring the fluorescence polarization, $P_M$, for a second aliquot of the lymphocytes that has been contacted with a mitogen selected from the group consisting of phytohaemagglutinin, concanavalin A, and pokeweed mitogen; and.
   (c) determining an SCM response ratio, $RR_{SCM}$, as the ratio of $P_S$ to $P_M$, whereby an $RR_{SCM}$ of less than about 0.9 indicates the presence of a malignancy in the donor of the lymphocytes.

18. A method for screening a blood sample for the presence of a malignancy in the body of the donor of the blood sample comprising:
   (a) separating potentially SCM-responding lymphocytes from the blood sample;
   (b) contacting the separated lymphocytes with the factor of any one of claims 1-14, thereby to stimulate the lymphocytes;
   (c) contacting the stimulated lymphocytes with a fluorogenic agent precursor for a sufficient time for the precursor to penetrate the lymphocytes for intracellular enzymatic hydrolysis to the fluorogenic compound, thereby generating stimulated fluor-containing lymphocytes;
   (d) exciting the stimulated fluor-containing lymphocytes with polarized light thereby causing the lymphocytes to fluoresce;
   (e) measuring the vertically polarized and horizontally polarized fluorescence emissions from the fluorescing lymphocytes for determining a polarization value for the fluorescing lymphocytes; and

(f) comparing the determined polarization value for the stimulated fluor-containing lymphocytes with the polarization value for a control aliquot of lymphocytes from the same donor which has been subjected to the operation of steps (a), (c), (d) and (e), but not (b), thereby to indicate the presence or absence of cancer in the body of the donor of the lymphocytes.

19. The method of claim 18 wherein the lymphocytes are excited with vertically polarized light and wherein the polarization values are determined in a fluorescence spectrophotometer in accordance with the relationship:

$$P = (I_V - GI_H)/(I_H + GI_H),$$

where $I_V$ and $I_H$ are the polarized fluorescence intensities in the vertical and horizontal planes, respectively, and G is a correction factor for the unequal transmission of the horizontal and vertical components of the polarized light through the optical system of the spectrophotometer.

20. The method of claim 18 or 19 wherein steps (b) and (c) occur simultaneously.

21. A method of imaging cancer cells comprising the steps of:

(a) labelling an antibody to the general cancer-associated SCM-recognition factor of any one of claims 1-14 with an imaging substance; and

(b) utilizing the labelled antibody to image cancer cells.

22. A method of determining the level of general cancer-associated SCM-recognition factor in a body fluid using an antibody to the general cancer-associated SCM-recognition factor of any one of claims 1-14 in an immunoassay.

23. The method of claim 22 wherein the immunoassay is a radioimmunoassay, a fluorescence immunoassay, an enzyme-linked immunosorbent assay, or a precipitation immunoassay including nephelometric assays and turbidimetric assays.

24. A process for producing a general cancer-associated SCM-recognition factor comprising the steps of:

(a) obtaining a body fluid from a donor afflicted with cancer;

(b) separating a first fraction of the body fluid comprising molecules having an apparent molecular weight greater than 1000 daltons from a second fraction comprising molecules having an apparent molecular weight of less than 1000 daltons; and

(c) purifying the second fraction to obtain a substantially pure general cancer-associated SCM-recognition factor.

25. The process of claim 24 wherein the body fluid is selected from peripheral blood, urine, and plasma.

26. The process of claim 24 or 25 wherein the separation of the second fraction from the first fraction is performed by filtration of the body fluid through an ultrafilter with a nominal 1000-dalton molecular weight cutoff.

27. The process of any one of claims 24-26 further comprising the step of desalting the second fraction by loading the second fraction on a gel filtration column with a fractionation range of from 0 to 700 daltons and capable of separating the salts therefrom, eluting the loaded material from the column with distilled water, and collecting that portion eluting at an elution volume between about 0.3 and about 0.5 times the total chromatographic bed volume, thereby increasing the specific activity of the general cancer-associated SCM-recognition factor in the collected desalted portion relative to the specific activity in the second fraction of claim 24.

28. The process of claim 27 further comprising the steps of:

(a) loading the collected desalted portion on a gel filtration column having a fractionation range of from about 1500 daltons to about 30,000 daltons;

(b) eluting the material loaded onto the column in step (a) from the column with a weak aqueous solution of an ammonium salt; and

(c) collecting that portion eluting at an elution volume between about 0.4 times and about 0.6 times the total chromatographic bed volume, thereby increasing the specific activity of the general cancer-associated SCM-recognition factor in the collected eluate relative to the specific activity in the collected desalted portion of claim 27.

29. The process of claim 28 further comprising the steps of:

(a) loading the collected eluate on a diethylaminoethyl cellulose anion-exchange column;

(b) eluting the material loaded onto the column in step (a) from the column with an increasing concentration of an ammonium salt; and

(c) collecting that portion eluting from the column at about 0.28 M to about 0.31 M of the ammonium salt, thereby increasing the specific activity of the general cancer-associated SCM-recognition factor in the collected eluate relative to the specific activity in the collected eluate of claim 28.

30. The process of claim 29 further comprising the steps of purifying the general cancer-associated SCM-recognition factor from the collected eluate of claim 29 to substantial homogeneity by reverse phase high-pressure liquid chromatography.

**Patentansprüche**

1. Im wesentlichen reiner, allgemein bei Krebs vorkommender, an der SCM erkennbarer Faktor, der im wesentlichen aus einem Peptid von niedrigem Molekulargewicht besteht, das durch Filter mit einer einem Molekulargewicht von 1000 Dalton entsprechenden Nenn-Trenngröße hindurchtritt und von Filtern mit einer einem Molekulargewicht von 500 Dalton entsprechenNenn-Trenngröße zurückgehalten wird und das die Aminosäurensequenz Lys-Pro-Phe besitzt, wobei der Faktor den Polirasationswert der intrazellulären Fluoreszenz von Lymphozyten mit veränderbarer SCM von an Krebs erkrankten Spendern bei Messung in der genormten SCM-Prüfung um mindestens zehn Prozent herabsetzt.

2. Faktor nach Anspruch 1, der infolge einer Reinigung im wesentlichen homogen ist.

3. Allgemein bei Krebs vorkommender, an der SCM nachweisbarer Faktor, der im wesentlichen nur Peptide enthält, die mindestens dreizehn Aminosäurereste besitzen, zu denen die Sequenz Phe-$R_1$-Lys-Pro-Phe-$R_2$-Phe-$R_3$-Met-$R_4$-$R_5$-$R_6$-$R_7$ gehört, wobei $R_1$ aus Asn und Gln ausgewählt ist, $R_2$, $R_3$ und $R_4$ unabhängig voneinander jeweils aus Val, Leu und Ile ausgewählt sind, $R_5$ aus Asp und Glu ausgewählt ist und $R_6$ und $R_7$ unabhängig voneinander jeweils aus Asn und Gln ausgewählt sind.

4. Faktor nach Anspruch 3, der geeignet ist, die SCM-Änderung von Lymphozyten mit veränderbarer SCM von von bösartigen Erkrankugen freien Spendern zu verändern, wenn der Faktor mit derartigen Lymphozyten mit veränderbarer SCM in Berührung gebracht wird, wobei die Lymphozyten bei der SCM-Prüfung auf bei Krebs vorkommende Antigene, aber nicht auf Mitogene ansprechen.

5. Faktor nach Anspruch 3 oder 4, der geeignet ist, die soontane Toxizität in vitro von Lymphozyten mit potentiell veränderbarer SCM für die Hymanmyeloidzellenlinie K 562 bei der Messung durch die Freisetzung von $^{51}$Cr um mindestens 90% zu vermindern.

6. Faktor nach einem der vorhergehenden Ansprüche, der annähernd die folgende Aminosäurenzusammensetzung hat: (Asx$_2$, Glx$_3$, Ser, His, Gly$_5$, Thr, Arg, Ala$_3$, Tyr, Met, Val$_3$, Phe$_3$, Ile, Leu$_3$, Lys$_2$).

7. Faktor nach einem der Ansprüche 1 bis 5, dadurch gekennzeichnet, daß ein aus sechzehn Aminosäuren bestehendes Segment in dem Faktor die Aminosäurensequenz Phe-Asn-Lys-Pro-Phe-Val-Phe-Leu-Met-Ile-Asp-Gln-Asn-Phe-Ser-Lys besitzt und dieses Segment nicht die endständige Aminogruppe des Faktors enthält.

8. Faktor nach einem der Ansprüche 1 bis 5, dadurch gekennzeichnet, daß ein aus fünfzehn Aminosäuren bestehendes Segment in dem Faktor die Aminosäurensequenz Phe-Asn-Lys-Pro-Phe-Val-Phe-Leu-Met-Ile-Asp-Gln-Asn-Thr-Lys besitzt, wobei des Segments nicht die endständige Aminogruppe des Faktors enthält.

9. Faktor nach einem der Ansprüche 1 bis 5, das im wesentlichen nur Peptide enthält, die mindestens fünfzehn Aminosäurereste besitzen, zu denen die Sequenz Phe-$R_1$-Lys-Pro-Phe-$R_2$-Phe-$R_3$-Met-$R_4$-$R_5$-$R_6$- $R_7$- $R_8$-Lys gehärt, wobei $R_5$ aus Ser und Thr ausgewählt ist.

10. Faktor nach Anspruch 9, der im wesentlichen nur Peptide enthält, die die Sequenz Phe- Asn-Lys-Pro-Phe-Val-Phe-Leu-Met-Ile-Asp-Gln-Asn-Thr-Lys besitzen.

11. Faktor nach einem der Ansprüche 1 bis 5, der im wesentlichen nur Peptide enthält, die mindestens sechzehn Aminosäurereste besitzen, zu denen die Sequenz Phe-$R_1$-Lys-Pro-Phe-$R_2$-Phe-$R_3$-Met-$R_4$-$R_5$-$R_5$-$R_7$-Phe-$R_6$-Lys gehört, wobei $R_6$ aus Ser und Thr ausgewählt ist.

12. Faktor nach Anspruch 11, der im wesentlichen nur Peqtide enthält, die die Sequenz Phe-Asn-Lys-Pro-Phe-Val-Phe-Leu-Met-Ile-Asq-Gln-Asn-Phe-Ser-Lys besitzen.

13. Faktor nach einem der vorhergehenden Ansprüche, der im wesentlichen nur ein Peptid enthält.

14. Peptid nach Anspruch 12 und 13, dadurch gekennzeichnet, daß durch 5 x 10$^2$ Femtogramm des Peqtids bei seiner Verwendung zum Provozieren von Lymphozyten mit veränderbarer SCM von krebskranken Spendern in der SCM-Prüfung der Polarisationswert um mindestens 30% herabgesetzt wird.

15. Verfahren zum Prüfen von von einem Säugetier als Spender stammenden Lymphozyten auf das Vorhandensein oder Nichtvorhandensein von bösartigen Erkrankungen beim Spender, mit folgenden Schritten:

(a) eine Suspension der Lymphozyten wird mit dem allgemein bei Krebs vorkommenden, an der SCM erkennbaren Faktor nach einem der vorhergehenden Ansprüche in Berührung gebracht, und

(b) die infolge der Berührung mit der Suspension der Lymphozyten bewirkte Herabsetzung der Strukturiertheit der Zytoplasmamatrix wird bestimmt.

16. Verfahren nach Anspruch 15, dadurch gekennzeichnet, daß die Herabsetzung der Strukturiertheit der Zytoplasmamatrix durch folgende Schritte bestimmt wird:

(a) für ein mit dem Faktor in Berührung gebrachtes Aliquot der Lymphozyten wird die Fluoreszenzpolarisation $P_S$ gemessen;

(b) für ein nicht mit dem Faktor in Berührung gebrachtes, zweites Aliquot der Lymphozyten wird die Fluo-

EP 0 357 637 B1

reszenzpolarisation $P_C$ gemessen und

(c) das Verhältnis von $P_S$ zu $P_C$ wird bestimmt, wobei ein Verhältnis von $P_S$ zu $P_C$ unter etwa 0,9 eine bösartige Erkrankung des Spenders der Lymphozyten anzeigt.

17. Verfahren nach Anspruch 15, dadurch gekennzeichnet, daß die Herabsetzung der Strukturiertheit der Zytoplasmamatrix durch folgende Schritte bestimmt wird:

(a) für ein mit dem Faktor in Berührung gebrachtes Aliquot der Lymphozyten wird die Fluoreszenzpolarisation $P_S$ gemessen;

(b) für ein zweites Aliquot der Lymphozyten, das mit einem Mitogen der Gruppe Phytohämagglutinin, Concanavalin A und "pokeweed"-Mitogen in Berührung gebracht worden ist, wird die Fluoreszenzpolarisation $P_M$ gemessen und

(c) als SCM-Veränderungsverhältnis $RR_{SCM}$ wird das Verhältnis von $P_S$ zu $P_M$ bestimmt, wobei ein $RR_{SCM}$ unter etwa 0,9 eine bösartige Erkrankung des Spenders der Lymphozyten anzeigt.

18. Verfahren zum Untersuchen einer Blutprobe auf eine bösartige Erkrankung des Körpers des Spenders der Blutprobe, in dem

(a) von der Blutprobe Lymphozyten mit veränderbarer SCM abgetrennt werden;

(b) die abgetrennten Lymphozyten mit dem Faktor nach einem der Ansprüche 1 bis 14 in Berührung gebracht und dadurch angeregt werden;

(c) die angeregten Lymphozyten solange mit einer Vorstufe eines fluorogenen Mittels in Berührung gebracht werden, daß die Vorstufe in die Lymphozyten eindringt und durch intrazelluläre enzymatische Hydrolyse in die fluorogene Verbindung überführt wird, so daß fluorhaltige angeregte Lymphozyten gebildet werden;

(d) die fluorhaltigen angeregten Kyphozyten mit polarisiertem Licht erregt und dadurch zur Fluoreszenz veranlaßt werden;

(e) zur Bestimmung eines Polarisationswertes für die fluoreszierenden Lymphozyten die von diesen emittierte, vertikal polarisierte bzw. horizontal polarisierte Fluoreszenz gemessen werden und

(f) zum Feststellen des Vorhandenseins oder Nichtvorhandenseins von Krebs im Körper des Spenders der Lymphozyten der so bestimmte Polarisationswert der fluorhaltigen angeregten Lymphozyten mit dem Polarisationswert eines Kontroll-aliquots von Lymphozyten verglichen wird, die den Schritten (a), (c), (d) und (e), aber nicht dem Schritt (b), unterworfen worden sind.

19. Verfahren nach Anspruch 18, dadurch gekennzeichnet, daß die Lymphozyten mit vertikal polarisiertem Licht erregt werden und daß die Polarisationswerte in einem Fluoreszenz-Spektralphotometer nach der Beziehung

$$P = (I_V - GI_H)/(I_H + GI_H)$$

bestimmt werden, in der $I_v$ und $I_h$ die Intensitäten der polarisierten Fluoreszenz in der Vertikal- bzw. Horizontalebene sind und G ein Korrekturfaktor zum Ausgleich des ungleichen Durchtritts der horizontalen und vertikalen Komponente des polarisierten Lichts durch die Optik des Spektralphotometers ist.

20. Verfahren nach Anspruch 18 oder 19, dadurch gekennzeichnet, daß die Schritte (b) und (c) gleichzeitig durchgeführt werden.

21. Verfahren zum Abbilden von Krebszellen mit folgenden Schritten:

(a) ein Antikörper gegen den allgemein bei Krebs vorkommenden, an der SCM erkennbaren Faktor nach einem der Ansprüche 1 bis 14 wird mit einer abbildbaren Substanz markiert und

(b) der markierte Antikörper wird zum Abbilden von Krebszellen verwendet.

22. Verfahren zum Bestimmen des Spiegels eines allgemein bei Krebs vorkommenden, an der SCM erkennbaren Faktors in einer Körperflüssigkeit unter Verwendung eines Antikörpers gegen den allgemein bei Krebs vorkommenden, an der SCM erkennbaren Faktor nach einem der Ansprüche 1 bis 14 in einem Immunoassay.

23. Verfahren nach Anspruch 22, dadurch gekennzeichnet, daß das Immunoassay ein Radioimmunoassay, ein Fluoreszenzimmunoassay, ein Assay mit einem mit Enzymen verbundenen Immunosorbens oder ein Ausfäll-Immunoassay, unter Einschluß von nephelometrischen und turbidimetrischen Assays, ist.

24. Verfahren zum Erzeugen eines allgemein bei Krebs vorkommenden, an der SCM erkennbaren Faktors mit folgenden Schritten:

(a) einem krebskranken Spender wird eine Körperflüssigkeit entnommen;

(b) eine erste Fraktion der Körperflüssigkeit mit Molekülen mit einem scheinbaren Molekulargewicht über 1000 Dalton wird von einer zweiten Fraktion mit Molekülen mit einem scheinbaren Molekulargewicht unter 1000 Dalton getrennt, und

(c) durch Reinigen der zweiten Fraktion wird ein im wesentlichen reiner, allgemein bei Krebs vorkommender, an der SCM erkennbarer Faktor gewonnen.

25. Verfahren nach Anspruch 24, dadurch gekennzeichnet, daß die Körperflüssigkeit aus peripherem Blut, Harn und Plasma ausgewählt wird.

26. Verfahren nach Anspruch 24 oder 25, dadurch gekennzeichnet, daß zum Abtrennen der zweiten von der ersten Fraktion die Körperflüssigkeit durch ein Ultrafilter mit einer einem Molekulargewicht von 1000 Dalton entsprechenden Nenn-Trenngröße filtriert wird.

27. Verfahren nach einem der Ansprüche 24 bis 26, in dem in einem weiteren Schritt zum Entsalzen der zweiten Fraktion diese auf eine Gelfiltriersäule mit einem Fraktionierbereich von 0 bis 700 Dalton aufgegeben wird, die zum Abtrennen der Salze von der zweiten Fraktion geeignet ist, worauf das Gut von der Säule eluiert und jener Teil gesammelt wird, der bei einem Elutionsvolumen vom etwa 0,3- bis zum etwa 0,5-fachen des Gesamtvolumens des für die Chromatographie verwendeten Bettes eluiert wird, so daß in dem gesammelten entsalzten Teil die spezifische Aktivität des allgemein bei Krebs vorkommenden, an der SCM erkennbaren Faktors gegenüber dessen spezifischer Aktivität in der zweiten Fraktion nach Anspruch 24 erhöht wird.

28. Verfahren nach Anspruch 27, mit folgenden weiteren Schritten:

(a) der gesammelte entsalzte Teil wird auf eine Gelfiltriersäule mit einem Fraktionierbereich von etwa 1500 Dalton bis etwa 30 000 Dalton aufgegeben;

(b) das im Schritt (a) aufgegebene Gut wird mit einer schwachen wäßrigen Lösung eines Ammoniumsalzes von der Kolonne eluiert; und

(c) es wird jener Teil gesammelt, der bei einem Elutionsvolumen von etwa 0,4-fachen bis zum etwa 0,6.fachen des Gesamtvolumens des für die Chromatographie verwendeten Bettel eluiert wird, wodurch die spezifische Aktivität des allgemein bei Krebs vorkommenden, an der SCM erkennbaren Faktors in dem gesammelten Eluat gegenüber der spezifischen Aktivität dieses Faktors in dem gesammelten entsalzten Teil nach Anspruch 27 erhöht wird.

29. Verfahren nach Anspruch 28 mit folgenden weiteren Schritten:

(a) das gesammelte Eluat wird auf eine Diethylaminoethyl-Cellulose-Anionenaustauschsäule aufgegeben;

(b) das im Schritt (a) auf die Säule aufgegebene Gut wird mit zunehmender Konzentration eines Ammoniumsalzes von der Säule eluiert; und

(c) der von etwa 0,28 M bis etwa 0,31 M Ammoniumsalz von der Säule eluierte Anteil wird gesammelt, so daß die spezifische Aktivität des allgemein bei Krebs vorkommenden, an der SCM erkennbaren Faktors in dem gesammelten Eluat gegenüber der spezifischen Aktivität dieses Faktors in dem nach Anspruch 28 gesammelten Eluat erhöht wird.

30. Verfahren nach Anspruch 29, in dem der aus dem nacht Anspruch 29 gesammelten Eluat gewonnene, allgemein bei Krebs vorkommende, an der SCM erkennbare Faktor durch Flüssigkeitschromatographie mit Phasenumkehr so gerrinigt wird, daß er im wesentlichen homogen ist.

## Revendications

1. Facteur associé au cancer reconnaissable par test ESMC (Etat de Structure de matrice cytoplasmique), sensiblement pur, constitué essentiellement d'un peptide de faible poids moléculaire passant à travers des filtres ayant un seuil théorique de coupure de poids moléculaire de 1000 daltons et retenu par des filtres ayant un seuil théorique de coupure de poids moléculaire de 500 daltons et incluant la séquence diacides aminés Lys-Pro-Phe, le facteur produisant une diminution d'au moins dix pour cent de la valeur de polarisation de fluorescence intracellulaire de lymphocytes répondeurs dans le test ESMC provenant de donneurs atteints de cancer, mesurée par le test standard d'ESMC.

2. Facteur selon la revendication 1 purifié à une homogénéité substantielle.

3. Facteur associé au cancer reconnaissable par test ESMC ne comprenant pour l'essentiel que des peptides ayant au moins treize résidus acides aminés incluant une séquence Phe-$R_1$-Lys-Pro-Phe-$R_2$-Phe-$R_3$-Met-$R_4$-$R_5$-$R_6$-$R_7$ , où $R_1$ est choisi parmi Asn et Gln, $R_2$, $R_3$ et $R_4$ sont choisis chacun indépendamment parmi Val, Leu et Ile, $R_6$ est choisi parmi Asp et Glu, et $R_6$ et $R_7$ sont choisis chacun indépendamment parmi Asn et Gln.

4. Facteur selon la revendication 3 ayant la propriété de modifier le changement d'ESMC de lymphocytes répondeurs dans le test ESMC obtenus auprès de donneurs exempts d'atteinte maligne lorsque le facteur est mis en contact avec de tels lymphocytes répondeurs dans le test ESMC, ce qui fait que les lymphocytes répondent dans le test ESMC aux antigènes associés au cancer mais non aux agents mitogènes après le contact.

5. Facteur selon la revendication 3 ou 4 ayant la propriété de diminuer la toxicité in vitro de lymphocytes potentiellement répondeurs dans le test ESMC envers la lignée cellulaire myéloïde humaine K 562 d'au moins 90%, mesurés par la libération de $^{51}$Cr.

6. Facteur selon l'une quelconque des revendications précédentes ayant la composition en acides amines approchée ($Asx_2$, $Glx_3$, Ser, His, $Gly_5$, Thr, Arg, $Ala_3$, Tyr, Met, $Val_3$, $Phe_3$, Ile, $Leu_3$, $Lys_2$).

7. Facteur selon l'une quelconque des revendications 1-5 dans lequel un segment à seize acides aminés

EP 0 357 637 B1

dans le facteur a la séquence d'acides aminés Phe-Asn-Lys-Pro-Phe-Val-Phe-Leu-Met-Ile-Asp-Gln-Asn-Phe-Ser-Lys, le segment n'incluant pas l'extrémité amino du facteur.

8. Facteur selon l'une quelconque des revendications 1-5 où un segment à quinze acides aminés dans le facteur à la séquence d'acides aminés Phe-Asn-Lys-Pro-Phe-Val-Phe-Leu-Met-Ile-Asp-Gln-Asn-Thr-Lys, le segment n'incluant pas l'extrémité amino du facteur.

9. Facteur selon l'une quelconque des revendications 1-5 ne comprenant pour l'essentiel que des peptides ayant au moins quinze résidus acides aminés incluant une séquence Phe-$R_1$-Lys-Pro-Phe-$R_2$-Phe-$R_3$-Met-$R_4$-$R_5$-$R_6$-$R_7$-$R_8$, où $R_5$ est choisi parmi Ser et Thr.

10. Facteur selon la revendication 9 ne comprenant pour l'essentiel que des peptides incluant une séquence Phe-Asn-Lys-Pro-Phe-Val-Phe-Leu-Met-Ile-Asp-Gln-Asn-Thr-Lys.

11. Facteur selon l'une quelconque des revendications 1-5 ne comprenant pour l'essentiel que des peptides ayant au moins seize résidus acides aminés incluant une séquence Phe-$R_1$-Lys-Pro-Phe-$R_2$-Phe-$R_3$-Met-$R_4$-$R_5$-$R_6$-$R_7$- Phe-$R_5$-Lys-, ou $R_8$ est choisi parmi Ser et Thr.

12. Facteur selon la revendication 11 ne comprenant pour l'essentiel que des peptides incluant une séquence Phe-Asn-Lys-Pro-Phe-Val-Phe-Leu-Met-Ile-Asp-Gln-Asn-Phe-Ser-Lys.

13. Facteur selon l'une quelconque des revendications précédentes ne contenant pour l'essentiel qu'un seul peptide.

14. Peptide selon les revendication 12 et 13 dans lequel $5 \times 10^2$ femtogrammes du peptide produisent une diminution de la valeur de polarisation d'au moins 30% dans le test ESMC lorsqu'on l'utilise pour provoquer des lymphocytes répondeurs dans le test ESMC provenant de donneurs atteints de cancer.

15. Procédé pour tester les lymphocytes obtenus à partir d'un donneur mammifère afin de rechercher la présence ou l'absence d'un état malin chez le donneur, comprenant les étapes de :

(a) mise en contact d'une suspension des lymphocytes avec le facteur associé au cancer reconnaissable par test ESMC de l'une quelconque des revendications précédentes ; et

(b) détermination de la diminution de l'état de structure de la matrice cytoplasmique des lymphocytes résultant de l'étape de mise en contact de la suspension des lymphocytes.

16. Procédé selon la revendication 15 dans lequel l'étape de détermination de la diminution de l'état de la structure de la matrice cytoplasmique comprend les étapes de :

(a) mesure de la polarisation le fluorescence, $P_S$, pour une partie aliquote des lymphocytes qui a été mise en contact avec le facteur ;

(b) mesure de la polarisation de fluorescence, $P_C$, pour une seconde partie aliquote des lymphocytes qui n'a pas été mise en contact avec le facteur; et

(c) détermination du rapport de $P_S$ à $P_C$, un rapport de $P_S$ à $P_C$ inférieur à environ 0,9 indiquant la présence d'un état malin chez le donneur des lymphocytes.

17. Procédé selon la revendication 15 dans lequel l'étape de détermination de la diminution de l'état de la structure de la matrice cytoplasmique comprend les étapes de ;

(a) mesure de la polarisation de fluorescence, $P_S$, pour une partie aliquote des lymphocytes qui a été mise en contact avec le facteur;

(b) mesure de la polarisation de fluorescence, $P_M$, pour une seconde partie aliquote des lymphocytes qui a été mise en contact avec un agent mitogène choisi dans le groupe constitué par la phytohémagglutinine, la concanavaline A, et l'agent mitogène de phytolaque; et

(c) détermination du rapport de réponse au test ESMC, $RRE_{SMC}$, comme étant le rapport de $P_S$ à $P_M$, où un $RRE_{SMC}$ inférieur à environ 0,9 indique la présence d'un état malin chez le donneur des lymphocytes.

18. Procédé pour déterminer par criblage dans un échantillon sanguin La présence d'un état malin dans le corps du donneur de l'échantillon de sang, dans lequel :

(a) on sépare les lymphocytes potentiellement répondeurs dans le test ESMC de l'échantillon sanguin ;

(b) on met en contact les lymphocytes séparés avec le facteur de l'une quelconque des revendications 1-14, pour stimuler ainsi les lymphocytes;

(c) on met en contact les lymphocytes stimulés avec un précurseur d'agent fluorogène pendant une durée suffisante pour que le précurseur pénètre les lymphocytes en vue d'une hydrolyse enzymatique intracellulaire aboutissant au composé fluorogéne, générant ainsi des lymphocytes stimulés contenant de la matière fluorescente ;

(d) on excite les lymphocytes stimulés contenant de la matière fluorescente avec de la lumière polarisée, provoquant ainsi la fluorescence des lymphocytes;

(e) on mesure les émissions de fluorescence polarisées verticalement et horizontalement en provenance des lymphocytes fluorescents pour déterminer une valeur de polarisation pour les lymphocytes fluorescents; et

(f) on compare la valeur de polarisation déterminée pour les lymphocytes stimulés contenant de la matière

34

fluorescente avec la valeur de polarisation pour une partie aliquote témoin de lymphocytes provenant du même donneur qui a été soumis à l'opération des étapes (a), (c), (d) et (e), mais non (b), pour indiquer ainsi la présence ou l'absence d'un cancer dans le corps du donneur des lymphocytes.

19. Procédé selon la revendication 18 dans lequel les lymphocytes sont excités avec de la lumière polarisée verticalement dans lequel les valeurs de polarisation sont déterminées dans un spectrophotomètre à fluorescence selon la relation;

$$P = (I_V - GI_H)/(I_H + GI_H),$$

dans laquelle $I_V$ et $I_H$ sont les intensités de fluorescence polarisée dans les plans vertical et horizontal, respectivement, et G est un facteur de correction de l'inégalité de transmission des composants horizontal et vertical de la lumière polarisée à travers le système optique du spectrophotomètre.

20. Procédé selon la revendication 18 ou 19 dans lequel les étapes (b) et (c) se déroulent simultanément.

21. Procédé pour représenter par une image des cellules cancéreuses comprenant les étapes de :

(a) marquage d'un anticorps contre le facteur associé au cancer reconnaissable par test ESMC de l'une quelconque des revendications 1-14 avec un agent marqueur ; et

(b) utilisation de l'anticorps marqué pour représenter par une image des cellules cancéreuses.

22. Procédé de détermination de la concentration d'un facteur associé au cancer reconnaissable par test ESMC dans un fluide corporel par l'emploi dans un immunodosage d'un anticorps contre le facteur associé au cancer reconnaissable par test ESMC de l'une quelconque des revendications 1-14.

23. Procédé selon la revendication 22 dans lequel l'immunodosage est un radioimmunodosage, un immunodosage de fluorescence, un dosage d'immunoabsorption liée à l'enzyme, ou un immunodosage de précipitation incluant des dosages néphélométriques et des dosages turbidimétriques.

24. Procédé de préparation d'un facteur associé au cancer reconnaissable par test ESMC comprenant les étapes de :

(a) obtention d'un fluide corporel à partir d'un donneur atteint de cancer ;

(b) séparation d'une première fraction du fluide corporel comprenant des molécules ayant un poids moléculaire apparent supérieur à 1000 daltons, d'une seconde fraction comprenant des molécules ayant un poids moléculaire apparent inférieur à 1000 daltons; et

(c) purification de la seconde fraction pour obtenir un facteur associé au cancer reconnaissable par test ESMC sensiblement pur.

25. Procédé selon la revendication 24 dans lequel le fluide corporel est choisi parmi le sang périphérique, l'urine et le plasma.

26. Procédé selon la revendication 24 ou 25 dans lequel la séparation de la seconde fraction de la première fraction s'effectue par filtration du fluide corporel à travers un ultrafiltre ayant un seuil théorique de coupure de poids moléculaire de 1000 daltons.

27. Procédé selon l'une quelconque des revendications 24-26 comprenant en outre l'étape de dessalage de la seconde fraction par chargement de la seconde fraction sur une colonne de filtration sur gel avec un intervalle de fractionnement allant de 0 à 700 daltons et capable d'en séparer les sels, élution de la matière chargée de la colonne avec de l'eau distillée, et collecte de la partie s'éluant à un volume d'élution compris entre environ 0,3 et environ 0,5 fois le volume total du lit chromatographique, augmentant ainsi l'activité spécifique du facteur associé au cancer reconnaissable par test ESMC dans la partie dessalée recueillie par rapport à son activité spécifique dans la seconde fraction selon la revendication 24.

28. Procédé selon la revendication 27 comprenant en outre les étapes de :

(a) charge de la partie desselée recueillie sur une colonne de filtration sur gel ayant un intervalle de fractionnement allant d'environ 1500 daltons à environ 30 000 daltons;

(b) élution de la matière chargée sur la colonne dans l'étape (a) de la colonne avec une solution aqueuse à concentration faible en un sel d'ammonium; et

(c) collecte de la partie s'éluant à un volume d'élution compris entre environ 0,4 fois et environ 0,6 fois le volume total du lit chromatographique, augmentant ainsi l'activité spécifique du facteur associé au cancer reconnaissable par test ESMC dans l'éluat recueilli par rapport à son activité spécifique dans la partie desselée recueillie selon la revendication 27.

29. Procédé selon la revendication 28 comprenant en outre les étapes de :

(a) charge de l'éluat recueilli sur une colonne d'échange d'anions en diéthylaminoéthyl-cellulose;

(b) élution de la matière chargée sur la colonne dans l'étape (a) de la colonne avec une concentration croissante d'un sel d'ammonium; et

(c) collecte de la partie s'éluant de la colonne à une concentration entre environ 0,28 M et environ 0,31 M en sel d'ammonium, augmentant ainsi l'activité spécifique du facteur associé au cancer reconnaissable par test ESMC dans l'éluat recueilli par rapport à son activité spécifique dans l'éluat recueilli selon la revendication 28.

30. Procédé selon la revendication 29 comprenant en outre les étapes de purification du facteur associé au cancer reconnaissable par test ESMC à partir de l'éluat recueilli selon la revendication 29 jusqu'à homogénéité importante par chromatographie liquide à haute pression à phase inverse.